(19) Europäisches Patentamt / European Patent Office / Office européen des brevets

(11) **EP 3 354 193 B1**

(12) **EUROPEAN PATENT SPECIFICATION**

(45) Date of publication and mention
of the grant of the patent:
**03.02.2021  Bulletin 2021/05**

(51) Int Cl.:
*A61B 5/02* (2006.01)      *A61B 5/0245* (2006.01)
*G16H 50/30* (2018.01)     *G16H 20/60* (2018.01)

(21) Application number: **15904699.4**

(22) Date of filing: **24.09.2015**

(86) International application number:
**PCT/JP2015/004861**

(87) International publication number:
**WO 2017/051442 (30.03.2017 Gazette 2017/13)**

(54) **INFORMATION PROCESSING DEVICE, DIGESTION RATIO ESTIMATING METHOD, INFORMATION PROCESSING SYSTEM AND DIGESTION RATIO ESTIMATING PROGRAM**

INFORMATIONSVERARBEITUNGSVORRICHTUNG, VERFAHREN ZUR KALKULATION DES VERDAUUNGSVERHÄLTNISSES, INFORMATIONSVERARBEITUNGSSYSTEM UND PROGRAMM ZUR KALKULATION DES VERDAUUNGSVERHÄLTNISSES

DISPOSITIF DE TRAITEMENT D'INFORMATIONS, PROCÉDÉ D'ESTIMATION DE TAUX DE DIGESTION, SYSTÈME DE TRAITEMENT D'INFORMATIONS, ET PROGRAMME D'ESTIMATION DE TAUX DE DIGESTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**01.08.2018  Bulletin 2018/31**

(73) Proprietor: **Fujitsu Limited**
**Kawasaki-shi, Kanagawa 211-8588 (JP)**

(72) Inventor: **OYA, Takuro**
**Kawasaki-shi**
**Kanagawa 211-8588 (JP)**

(74) Representative: **Haseltine Lake Kempner LLP**
**138 Cheapside**
**London EC2V 6BJ (GB)**

(56) References cited:
JP-A- H10 504 739      JP-A- 2003 173 375
JP-A- 2007 048 180     JP-A- 2010 510 010
JP-A- 2012 045 191     JP-A- 2013 220 110
US-A- 5 398 688        US-A1- 2006 064 037

- OUCHI K ET AL: "Lifeminder: A wearable healthcare support system with timely instruction based on the user's context", ADVANCED MOTION CONTROL, 2004 8TH IEEE INTERNATIONAL WORKSHOP ON. AMC KAWASAKI, JAPAN MARCH 25-28, 2004, PISCATAWAY, NJ, USA,IEEE, 25 March 2004 (2004-03-25), pages 445-450, XP010705746, DOI: 10.1109/AMC.2004.1297910 ISBN: 978-0-7803-8300-5
- KIMIYO YAMAGUCHI: 'Sesshoku ga Myakuhaku To ni Oyobosu Eikyo -Kenkosha 12-mei ni Tsuite' JOURNAL OF JAPANESE SOCIETY OF NURSING RESEARCH vol. 1, no. 2, 20 June 1978, pages 14 - 18, XP0155370968

**Description**

FIELD

The embodiments discussed herein are related to an information processing device, a digestion rate estimation method, an information processing system, and a digestion rate estimation program.

BACKGROUND

In recent years, services for sensing a state or behavior of a user and distributing information based on the state or behavior to the user have been provided by service providers.

For example, a service for providing beneficial information to a user based on the rate of digestion of ingested food has been disclosed ("Display of calories of photographed food and determination of degree of hunger based on breath - Docomo diet support service", [online], ITmedia Inc., [Searched on July 6, 2015], Internet <URL: http://www.itmedia.co.jp/mobile/articles/1110/06/news128.html>). In this service, a technique for measuring acetone concentration from exhaled breath and estimating a digestion rate (or the degree of hunger) from the acetone concentration is used. A user may know the timing of having meals and exercising based on the estimated degree of hunger via a mobile information terminal. As related-art documents, Japanese Unexamined Patent Publication (Translation of PCT application) No. 10-504739, Japanese Laid-open Patent Publication No. 2004-138, Japanese Laid-open Patent Publication No. 2009-201805, Japanese Laid-open Patent Publication No. 2011-115508, and Japanese Laid-open Patent Publication No. 2008-61790 have been disclosed.

US5398688 discloses a method for monitoring food intake for an individual and providing an indication when the food intake exceeds a predetermined allowable amount.

US2006/064037 discloses a system for detecting non-verbal acoustic energy generated by a subject.

Ouchi, K., et al. "LifeMinder: a wearable healthcare support system with timely instruction based on the user's context." In The 8th IEEE International Workshop on Advanced Motion Control 2004 (AMC2004), pages 445-450, discloses a wearable healthcare support system that provides patients with timely instructions in accordance with their current context.

SUMMARY

[TECHNICAL PROBLEM]

A device that measures the aforementioned acetone concentration is not small enough to usually wear the device. Every time acetone concentration is monitored, a task of removing acetone remaining in the device is performed. It is, therefore, difficult to monitor acetone concentration and provide a service based on the rate of digestion of ingested food to a user in real time.

According to an aspect, an object of the present disclosure is to provide an information processing device, a digestion rate estimation method, and a digestion rate estimation program that enable a service based on the rate of digestion of ingested food to be provided to a user in real time.

[SOLUTION TO PROBLEM]

Aspect of the invention provide an information processing device according to claim 1, a digestion rate estimation method according to claim 9, an information processing system according to claim 10, and a digestion rate estimation program according to claim 11.

[ADVANTAGEOUS EFFECTS OF INVENTION]

In an aspect, it is possible to provide an information processing device, a digestion rate estimation method, and a digestion rate estimation program that enable a service based on the rate of digestion of ingested food to be provided to a user in real time.

BRIEF DESCRIPTION OF DRAWINGS

FIG. 1 is a diagram illustrating an example of an information processing system according to a first embodiment;

FIG. 2 is a diagram illustrating an example of a hardware configuration of an information processing device according to the first embodiment;

FIG. 3 is a diagram illustrating an example of a change in a heart rate over time during a time period from time before a meal to time after the meal.

FIG. 4 is a first flowchart of an example of a digestion rate estimation method to be executed by the information processing device;

FIG. 5 is a diagram illustrating an example of an initial state of a heart rate management table;

FIG. 6 is a diagram illustrating an example in which information is stored in the heart rate management table;

FIG. 7 is a diagram illustrating an example of an initial state of a meat time management table;

FIG. 8 is a diagram illustrating an example in which information is stored in the meal time management table in S102;

FIG. 9 is a diagram illustrating an example of an initial state of a characteristic amount management table;

FIG. 10 is a diagram illustrating an example in which information is stored in the characteristic amount management table in S103;

FIG. 11 is a diagram illustrating an example in which information is stored in the characteristic amount management table in S104;

FIG. 12 is a diagram describing a method for estimating a digestion rate using a primary approximate straight line;

FIG. 13 is a diagram illustrating an example in which information is stored in the characteristic amount management table in S105;

FIG. 14 is a diagram illustrating an example in which information is stored in the characteristic amount management table in S106;

FIG. 15 is a diagram illustrating an example in which information is stored in the characteristic amount management table in S108;

FIG. 16 is a second flowchart of the example of the digestion rate estimation method to be executed by the information processing device;

FIG. 17 is a diagram illustrating an example of an initial state of a digestion rate estimation management table;

FIG. 18 is a diagram illustrating an example in which information is stored in the digestion rate estimation management table in S110;

FIG. 19 is a diagram illustrating an example in which information is stored in the digestion rate estimation management table multiple times;

FIG. 20 is a diagram illustrating a modified example of the flowchart illustrated in FIG. 16;

FIG. 21 is a diagram illustrating an example of an information processing system according to a second embodiment;

FIG. 22 is a first flowchart of an example of a digestion rate estimation method to be executed by an information processing device according to the second embodiment;

FIG. 23 is a second flowchart of the example of the digestion rate estimation method to be executed by the information processing device according to the second embodiment;

FIG. 24 is a diagram illustrating an example of an information processing system according to a third embodiment;

FIG. 25 is a diagram illustrating an example of an information processing system according to a fourth embodiment;

FIG. 26 is a first flowchart of an example of a digestion rate estimation method to be executed by an information processing device according to the fourth embodiment;

FIG. 27 is a diagram illustrating an example of a change rate and digestion rate correspondence table;

FIG. 28 is a diagram illustrating a profile of the rate of change of the heart rate in order to describe the change rate and digestion rate correspondence table illustrated in FIG. 27; and

FIG. 29 is a second flowchart of the example of the digestion rate estimation method to be executed by the information processing device according to the fourth embodiment.

DESCRIPTION OF EMBODIMENTS

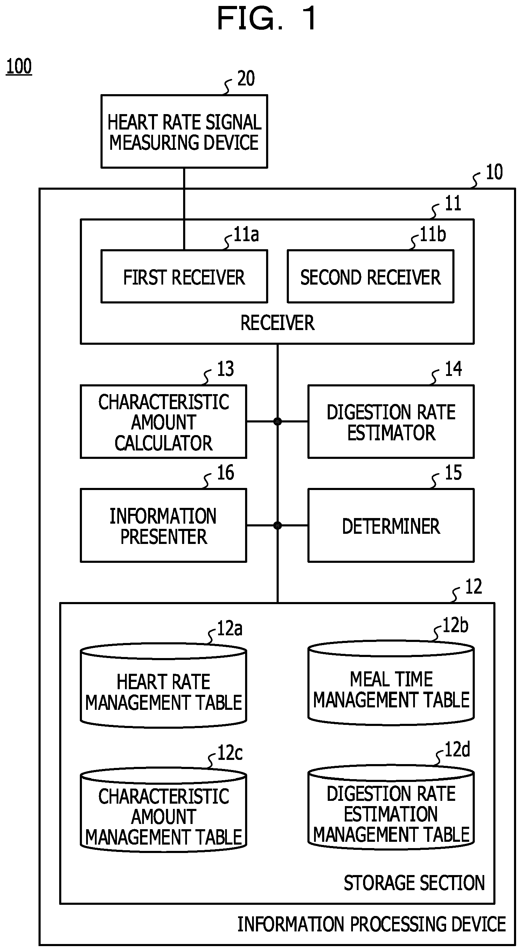

Hereinafter, embodiments are described with reference to FIGs. 1 to 29.

First Embodiment

FIG. 1 is a diagram illustrating an example of an information processing system 100 according to a first embodiment. As illustrated in FIG. 1, the information processing system 100 includes an information processing device 10 and a heart rate signal measuring device 20.

The information processing device 10 is a computer carried by a user who receives a service based on the rate of digestion of food ingested in the stomach of the user. The information processing device 10 estimates the digestion rate from the heart rate (pulse), received from the heart rate signal measuring device 20, of the user and provides the service to the user based on the result of the estimation. For example, the information processing device 10 is the computer such as a smartphone, a mobile phone, a tablet terminal, a laptop computer (personal computer), or a wearable computer. The wearable computer is wearable on and able to be carried by the user and is, for example, a wristwatch device.

The heart rate signal measuring device 20 is configured to measure the heart rate (pulse) and is, for example, a wristwatch heart rate meter. The heart rate signal measuring device 20 may measure the heart rate of the user wearing the heart rate signal measuring device 20 at predetermined time intervals. The heart rate signal measuring device 20 may transmit information of the measured heart rate to the information processing device 10.

The information processing system 100 may be achieved as a wearable computer including the information processing device 10 and the heart rate signal measuring device 20. In the case where the information processing system 100 is achieved by the wearable computer, the user may easily wear the information processing system 100.

Functions of sections forming the information processing device 10 are described below.

As illustrated in FIG. 1, the information processing device 10 includes a receiver 11, a storage section 12, a characteristic amount calculator 13, a digestion rate estimator 14, a determiner 15, and an information presenter 16.

The receiver 11 includes a first receiver 11a and a second receiver 11b. The first receiver 11a receives, from the heart rate signal measuring device 20, multiple heart rate signal information items of the heart rate measured at the predetermined time intervals. The heart rate signal information items are information in which the heart rate of the user is associated with time when the heart rate has been measured. The second receiver 11b receives, from the user, information of meal start time and meal end time. The meal start time is time when the user has started a meal. The meal end time is time when the user has ended the meal.

The storage section 12 is hardware for storing a program and data to be used for processes to be executed by the information processing device 10. For example, in the storage section 12, a heart rate management table 12a, a meal time management table 12b, a characteristic amount management table 12c, a digestion rate estimation management table 12d, and the like, which are used for the processes to be executed by the information processing device 10, are stored. Details of the tables are described later.

The storage section 12 is, for example, a hard disk drive (HDD), a solid state drive (SSD), a random access memory (RAM), a flash memory, or a read only memory (ROM) or may be a combination of two or more of the HDD, the SSD, the RAM, the flash memory, and the ROM. For example, the HDD, the SSD, the RAM, and a NAND-type flash memory may be used to store the data. A NOR-type flash memory and the ROM may be used to store the program (digestion rate estimation program or the like). The storage section 12 may be composed of multiple storage devices, depending on the use of the storage section 12, the capacity of the storage section 12, and the like.

The characteristic amount calculator 13 calculates various parameters to be used to calculate the digestion rate, based on the multiple heart rate signal information items received from the heart rate signal measuring device 20 and the information of the meal start time and meal end time of the user. The various parameters are hereinafter referred to as "characteristic amounts". Details of the various characteristic amounts and methods for calculating the various characteristic amounts are described later.

The digestion rate estimator 14 estimates the digestion rate based on the various characteristic amounts calculated by the characteristic amount calculator 13.

The determiner 15 executes various determination processes among the processes to be executed by the information processing device 10.

The information presenter 16 presents information to the user if the determiner 15 determines that the information on the digestion rate is to be presented to the user.

Next, a hardware configuration of the information processing device 10 is described.

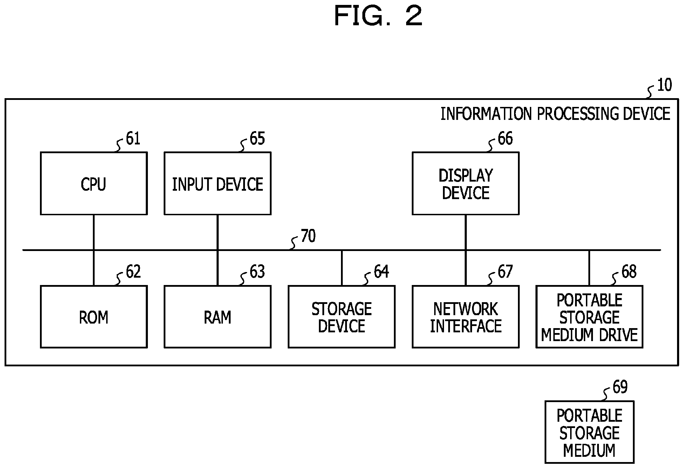

FIG. 2 is a diagram illustrating an example of the hardware configuration of the information processing device 10 according to the first embodiment. As illustrated in FIG. 2, the information processing device 10 includes a central processing unit (CPU) 61, a ROM 62, a RAM 63, a storage device 64, an input device 65, a display device 66, a network interface 67, a portable storage medium drive 68, and the like.

The CPU 61 is hardware that manages or executes the processes of the information processing device 10. An example of the CPU 61 is a micro processing unit (MPU). The CPU 61 is an example of the receiver 11, the characteristic amount calculator 13, the digestion rate estimator 14, the determiner 15, and the information presenter 16.

The ROM 62, the RAM 63, and the storage device 64 are hardware for storing the program and the data to be used for the processes to be executed by the CPU 61. The storage device 64 is, for example, an HDD. The ROM 62, the RAM 63, and the storage device 64 are an example of the storage section 12.

The input device 65 is hardware to be used for the user to enter various types of information. The input device 65 is, for example, a touch panel, a keyboard, a mouse, or the like. If the input device 65 is the touch panel, the touch panel is mounted on the display device 66 described later so that the touch panel overlaps the display device 66.

The display device 66 is configured to display an image. The display device 66 is, for example, a liquid crystal display, a plasma display, an organic electroluminescence display, or the like.

The network interface 67 is hardware that communicates with an external device via a network.

The constituent sections of the information processing device 10 are connected to a bus 70. In the information processing device 10, functions of the information processing device 10 are achieved by causing a processor such as the CPU 61 to execute a program (including the digestion rate estimation program) stored in the ROM 62 or the storage device 64 or execute the program (including the digestion rate estimation program) read by the portable storage medium drive 68 from the portable storage medium 69. The program may be loaded into the RAM 63 and executed by the processor such as the CPU 61.

Next, a digestion rate estimation method to be executed by the information processing device 10 is described. The inventor has measured a change in the heart rate during a time period from time before the meal and to time after the meal and found that a profile of the change in the heart rate has a specific trend.

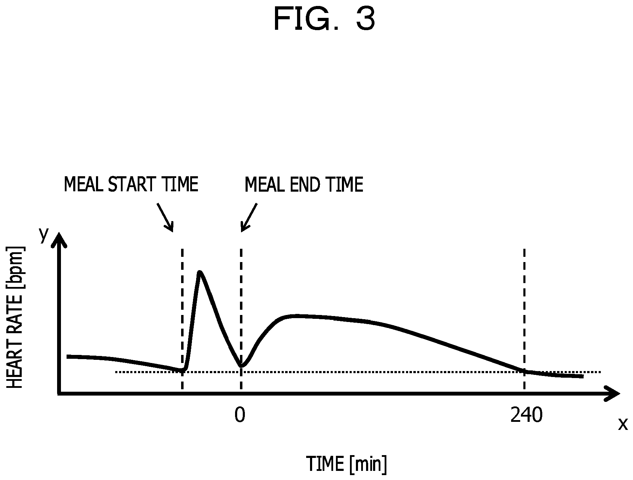

FIG. 3 is a diagram illustrating an example of the change in the heart rate over time during the time period from the time before the meal and to the time after the meal. An ordinate (y axis) indicates the heart rate, while an abscissa (x axis) indicates a time axis indicating that the time when the user has ended the meal is 0 minutes. In FIG. 3, meal start time indicates the time when the user has started the meal, and meal end time indicates the time when the user has ended the meal. In the following description, the profile of the change in the heart rate over time is referred to as "heart rate profile" in some cases. As illustrated in FIG. 3, when the user starts the meal, the heart rate rapidly increases. After the rapid increase, the heart rate decreases and becomes a local minimal value at the meal end time. The local minimal value is slightly higher than the heart rate at the meal start time. A change in the heart rate during the meal is estimated to occur due to a masticatory activity or a swallowing activity during the ingestion of food.

After the user ends the meal, the heart rate increases again and becomes a peak at certain time and gradually decreases after the certain time. Then, the heart rate returns to the value at the meal start time and becomes stable. It is estimated that the change in the heart rate after the meal is caused by a digestive activity in the stomach or the like and that the heart rate returns to a level before the meal due to the stop of the digestive activity. In the example illustrated in FIG. 3, the heart rate returns to the level before the meal after 240 minutes elapse after the meal end time. The time when the heart rate returns to the level before the meal varies depending on details (the type of the meal, the amount of the meal, and the like) of the meal. In the first embodiment, the digestion rate is estimated in real time using the aforementioned trend of the change in the heart rate.

Next, the flow of a process, to be executed by the information processing device 10, of estimating the digestion rate and presenting the information to the user based on the result of the estimation is described.

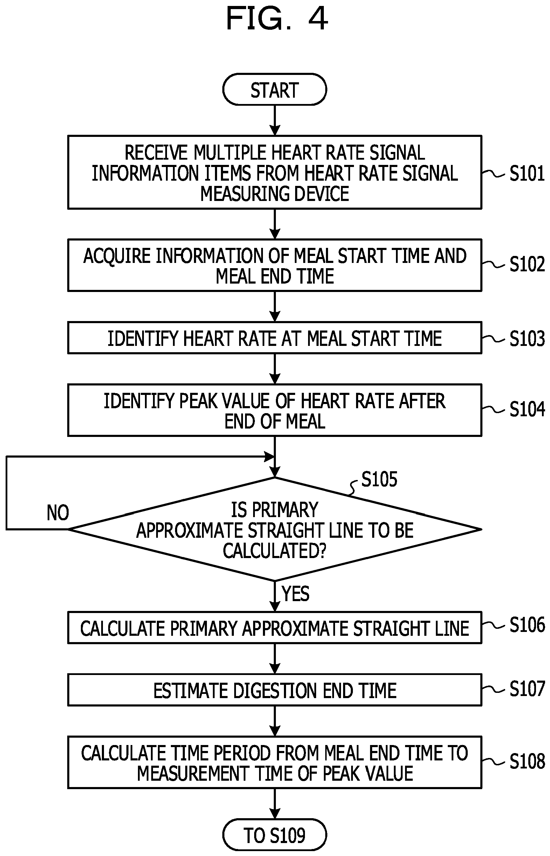

FIG. 4 is a flowchart of an example of the digestion rate estimation method to be executed by the information processing device 10.

First, the first receiver 11a receives multiple heart rate signal information items from the heart rate signal measuring device 20 (in S101). In S101, the first receiver 11a acquires the multiple heart rate signal information items from the heart rate signal measuring device 20 via wireless communication at predetermined time intervals, for example. Then, the first receiver 11a causes the acquired multiple heart rate signal information items to be stored in the heart rate management table 12a included in the storage section 12.

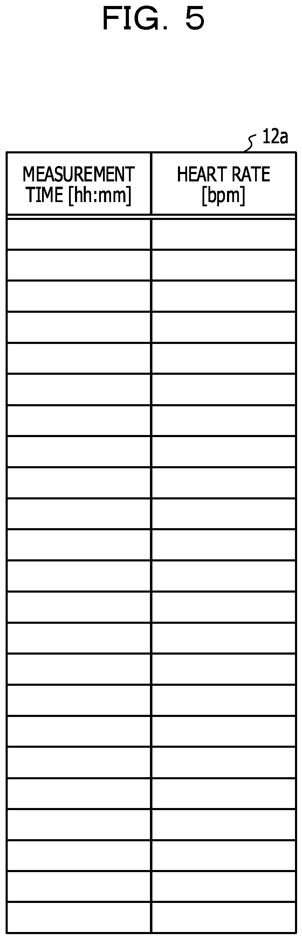

FIG. 5 is a diagram illustrating an example of an initial state of the heart rate management table 12a. The heart rate management table 12a includes a heart rate item and an item for time when the heart rate has been measured.

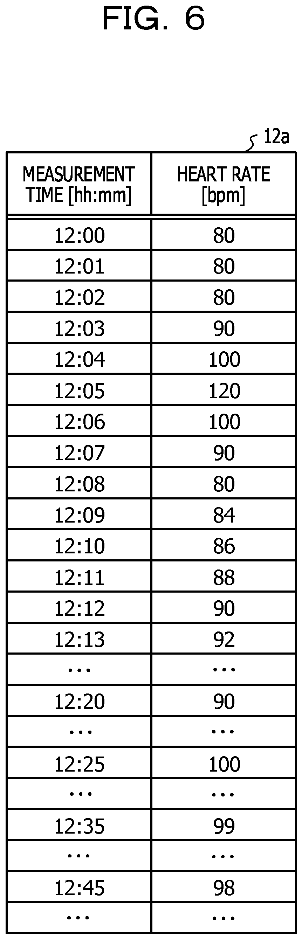

FIG. 6 is a diagram illustrating an example in which the information is stored in the heart rate management table 12a. In the example illustrated in FIG. 6, "12:00" indicates 12 o'clock 00 minutes, and information of the heart rate measured at time intervals of 1 minute is stored in the heart rate management table 12a. The measurement of the heart rate may be started from 0 o'clock 00 minutes each day, and information stored in the heart rate management table 12a may be managed for each day and may be deleted for each day. This may suppress an increase in a storage region included in the storage section 12 and to be used to store the heart rate management table 12a.

Return to FIG. 4. After the process of S101, the second receiver 11b acquires information of the meal start time and the meal end time upon sequentially receiving heart rate signal information items from the heart rate signal measuring device 20 (in S102). For example, in S102, the user uses the input device 65 to enter the information of the meal start time and the meal end time. Thus, the second receiver 11b may receive the information of the meal start time and the meal end time.

Alternatively, the information processing device 10 may use a method (refer to Japanese Laid-open Patent Publication No. 2011-115508) for identifying the meal start time and the meal end time by causing an acceleration sensor to detect a motion of a forearm upon the ingestion of food as a method for acquiring the information of the meal start time and the meal end time. Alternatively, the information processing device 10 may use a method (refer to Japanese Laid-open Patent Publication No. 2008-7-61790) for identifying the meal start time and the meal end time by using a body sound microphone to detect a frequency pattern specific to mastication.

In S102, the second receiver 11b causes the acquired information of the meal start time and the meal end time to be stored in the meal time management table 12b included in the storage section 12.

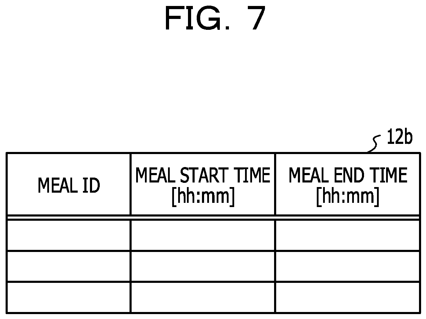

FIG. 7 is a diagram illustrating an example of an initial state of the meal time management table 12b. As illustrated in FIG. 7, the meal time management table 12b includes a meal identifier (ID) item indicating identifiers identifying meal events, a meal start time item, and a meal end time item and is used to manage meal start time and meal end time for each of meal IDs. Upon receiving information of meal start time and meal end time, the second receiver 11b gives a new meal ID for the received information and registers the new meal ID in the meal time management table 12b.

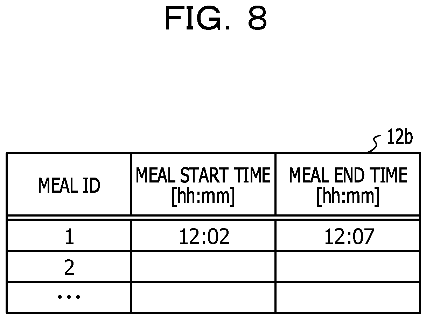

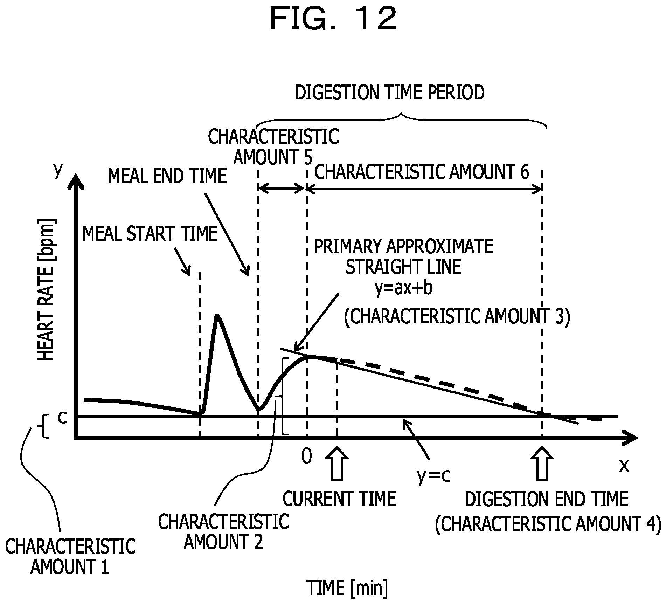

FIG. 8 is a diagram illustrating an example in which the information is stored in the meal time management table 12b in S102. In the example illustrated in FIG. 8, 12:02 is registered as meal start time for a meal for which a meal ID = 1 is given, while 12:07 is registered as meal end time for the meal for which the meal ID = 1 is given. The flow of the process is described below using the aforementioned information.

After the process of S102, the characteristic amount calculator 13 identifies the heart rate at the meal start time (in S103). Specifically, the characteristic amount calculator 13 references the heart rate management table 12a illustrated in FIG. 6 and identifies a heart rate associated with the meal start time registered in the heart rate management table 12a. For example, the meal start time associated with the meal ID = 1 is 12:02 in the example illustrated in FIG. 8. Referring to FIG. 6, a heart rate associated with 12:02 is 80 beats per minute (bpm). The characteristic amount calculator 13 causes information of the identified heart rate of 80 bpm to be stored in the characteristic amount management table 12c.

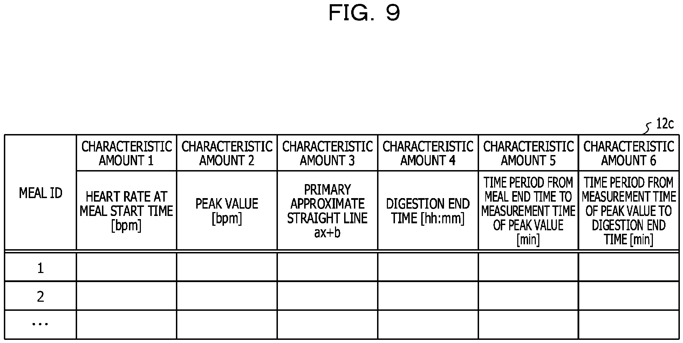

FIG. 9 is a diagram illustrating an example of an initial state of the characteristic amount management table 12c. As illustrated in FIG. 9, six types of characteristic amounts 1 to 6 may be stored for each meal ID in the characteristic amount management table 12c. In the first embodiment, the heart rate at the meal start time is referred to as "characteristic amount 1", and a peak value or the maximum value of the heart rate after the meal end time is referred to as "characteristic amount 2". In addition, a primary approximate straight line of the heart rate profile after the time (hereinafter also referred to as measurement time of the peak value) when the peak value is measured is referred to as "characteristic amount 3", and digestion end time is referred to as "characteristic amount 4". Furthermore, a time period from the meal end time to the time when the peak value is measured is referred to as "characteristic amount 5", and a time period from the time when the peak value is measured to the digestion end time is referred to as "characteristic amount 6". Details of the characteristic amounts 2 to 6 are described later.

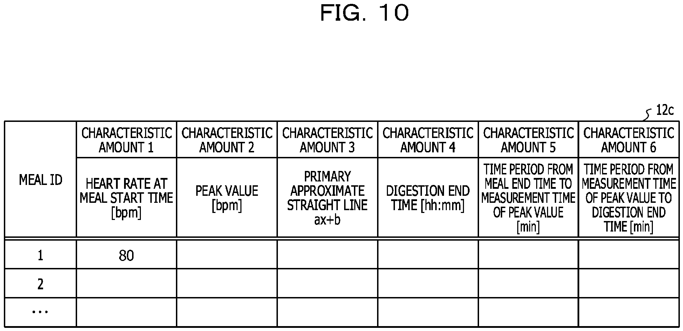

FIG. 10 is a diagram illustrating an example in which the information is stored in the characteristic amount management table 12c in S103. As illustrated in FIG. 10, the characteristic amount calculator 13 causes the information of the heart rate of 80 bpm identified in the process of S103 to be stored in a cell corresponding to the meal ID = 1 and included in a "characteristic amount 1" item.

Subsequently, the characteristic amount calculator 13 identifies the peak value of the heart rate after the meal end time (in S104). For example, if the meal end time is 12:07 as illustrated in FIG. 8, and the heart rate management table 12a at the start of the process of S104 is in a storage state illustrated in FIG. 6, the characteristic amount calculator 13 references the heart rate management table 12a illustrated in FIG. 6 and identifies that a peak value of the heart rate in a time zone after the end of the meal is 100 bpm measured at time of 12:25.

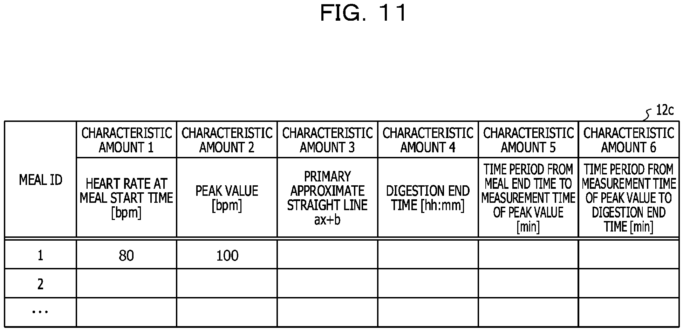

FIG. 11 is a diagram illustrating an example in which the information is stored in the characteristic amount management table 12c in S104. As illustrated in FIG. 11, the characteristic amount calculator 13 causes the identified peak value of 100 bpm to be stored in a cell corresponding to the meal ID = 1 and included in a "characteristic amount 2" item.

Subsequently, the determiner 15 determines whether or not the primary approximate straight line is to be calculated (in S105). A background of the process of S105 is described below.

As described with reference to FIG. 3, the heart rate profile has a shape indicating that, after the heart rate increases again from the end of the meal and reaches the peak value, the heart rate gradually decreases to the value at the meal start time. However, if the digestive activity occurs, a heart rate profile depicted based on multiple heart rate signal information items acquired until the digestive activity occurs indicates that the heart rate does not reach the value at the meal start time, and the time (digestion end time) when the digestive activity ends is not clear. In the first embodiment, the primary approximate straight line is used to identify the digestion end time using the heart rate profile indicating that the heart rate does not reach the value at the meal start time.

FIG. 12 is a diagram describing a method for estimating the digestion rate using the primary approximate straight line. FIG. 12 illustrates a profile depicted using a solid line based on heart rate signal information items acquired by the first receiver 11a. An ordinate (y axis) indicates the heart rate, and an abscissa (x axis) indicates a time axis indicating that the time when a peak value of the heart rate is measured after the end of the meal is 0 minutes. In addition, a right end of the profile indicated by the solid line indicates the magnitude of the heart rate measured at the current time. Then, a profile depicted based on unknown heart rate signal information items that are yet to be acquired by the first receiver 11a is indicated by a broken line.

As illustrated in FIG. 12, the information processing device 10 approximates, to the primary approximate straight line, a change in the heart rate measured during a predetermined time zone after the time when the peak value is measured. Since the x axis is the time axis and the y axis indicates the heart rate, the primary approximate straight line may be expressed by $y = ax + b$, where a indicates the inclination of the heart rate profile indicating a downward trend after the time when the peak value of the heart rate is measured, and b indicates the heart rate at the time when the peak value of the heart rate is measured, as described later. If the value of the heart rate at the meal start time is c, and the time when the heart rate returns to c after the meal is digestion end time, the digestion end time may be calculated by calculating an x value of an intersection of the straight line of y = ax + b with a line of y = c. The first embodiment assumes that a time zone of approximately 10 minutes to 20 minutes before the current time and after the time when the peak value is measured is the aforementioned "predetermined time zone". If information of the heart rate during the time zone is already acquired, the information processing device 10 calculates the primary approximate straight line obtained by approximating the change in the heart rate measured after the time when the peak value is measured. S105 is a process of determining whether or not heart rate signal information items sufficient to calculate the primary approximate straight line are already acquired.

In S105, the determiner 15 sets, as a set time period, 20 minutes or a time period that has elapsed after the peak value was measured, for example.

Subsequently, the determiner 15 references the heart rate management table 12a illustrated in FIG. 6 and determines whether or not all heart rate signal information items measured in the time zone of 20 minutes from the time when the peak value is measured are already stored. If all the heart rate signal information items are not stored, stored heart rate signal information items are not sufficient to calculate the primary approximate straight line, and the determiner 15 determines that the primary approximate straight line is not to be calculated (No in S105). If the answer to S105 is negative, 20 minutes already elapse from the time when the peak value is measured, and the determiner 15 stands by until heart rate signal information items sufficient to calculate the primary approximate straight line are acquired. Specifically, the determiner 15 repeatedly executes the process of S105.

If all the heart rate signal information items are already stored, the heart rate signal information items sufficient to calculate the primary approximate straight line are already acquired. Thus, the determiner 15 determines that the primary approximate straight line is to be calculated (Yes in S105), and the characteristic amount calculator 13 calculates the primary approximate straight line (in S106). In S106, the characteristic amount calculator 13 extracts, from the heart rate management table 12a, multiple heart rate signal information items measured in the predetermined zone from the time when the peak value is measured. Then, the characteristic amount calculator 13 uses information of the peak value stored in the "characteristic amount 2" item of the characteristic amount management table 12c and the extracted heart rate signal information items to calculate the primary approximate straight line that is a heart rate function using time as a variable.

For example, as illustrated in FIG. 12, if the time when the peak value is measured is the origin on the time axis (x axis), the characteristic amount 2 or the peak value of 100 bps of the heart rate is equal to the value of the y intercept of the primary approximate straight line. Referring to the heart rate management table 12a illustrated in FIG. 6, the time when the peak value of 100 bps is measured is 12:25. If the time period set in S105 is 20 minutes, the characteristic amount calculator 13 extracts multiple heart rate signal information items measured in the time zone from 12:25 to 12:45 that is 20 minutes after 12:25. Then, the characteristic amount calculator 13 calculates the inclination of the primary approximate straight line of the heart rate based on values of the extracted multiple heart rate signal information items and obtains -0.1. As a result, when x minutes elapse after the time when the peak value is measured, the characteristic amount calculator 13 may obtain y = -0.1x + 100 as the primary approximate straight line of the heart rate.

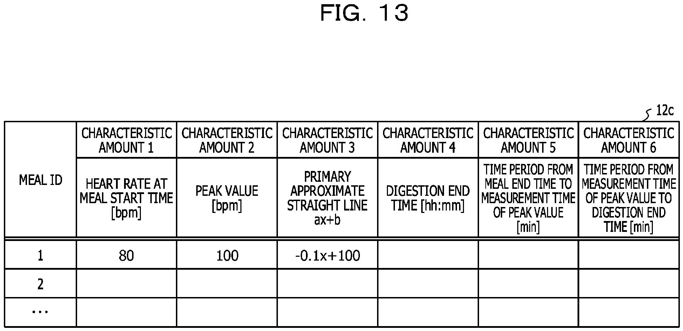

FIG. 13 is a diagram illustrating an example in which the information is stored in the characteristic amount management table 12c in S105. As illustrated in FIG. 13, the characteristic amount calculator 13 causes information of the calculated primary approximate straight line of y = -0.1x + 100 to be stored in a cell corresponding to the meal ID = 1 and included in the "characteristic amount 3" item.

Return to FIG. 4. After the process of S106, the characteristic amount calculator 13 uses the heart rate measured at the meal start time and identified in S103 and the primary approximate straight line calculated in S105 to calculate a time period from the measurement time of the peak value to the digestion end time and identifies the digestion end time (in S107).

The characteristic amount calculator 13 uses the primary approximate straight line, which is the profile obtained during the decrease in the heart rate, to calculate an x value corresponding to the time when the heart rate returns to the value at the meal start time. In this method, the characteristic amount calculator 13 may identify the digestion end time.

Referring to the "characteristic amount 1" item of the characteristic amount management table 12c illustrated in FIG. 13, the heart rate at the meal start time is 80 bpm. The characteristic amount calculator 13 calculates an x value satisfying 80 = -0.1x + 100 based on the primary approximate straight line stored in the "characteristic amount 3" item. As a result, x = 200 is obtained and the characteristic amount calculator 13 may identify that the digestive activity ends after the elapse of 200 minutes after the time when the peak value is measured. The time when the peak value is measured is 12:25 based on the heart rate management table 12a illustrated in FIG. 6. Thus, the digestion end time is 200 minutes after 12:25 or is calculated to be 15:45.

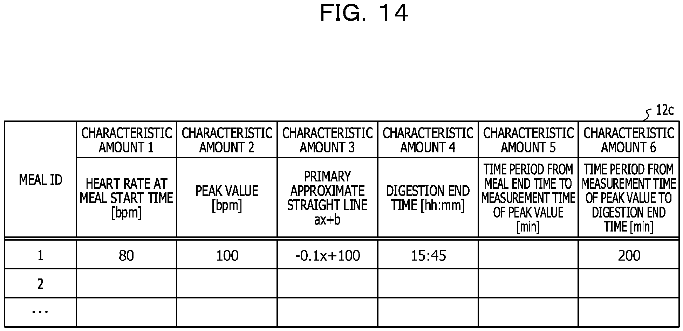

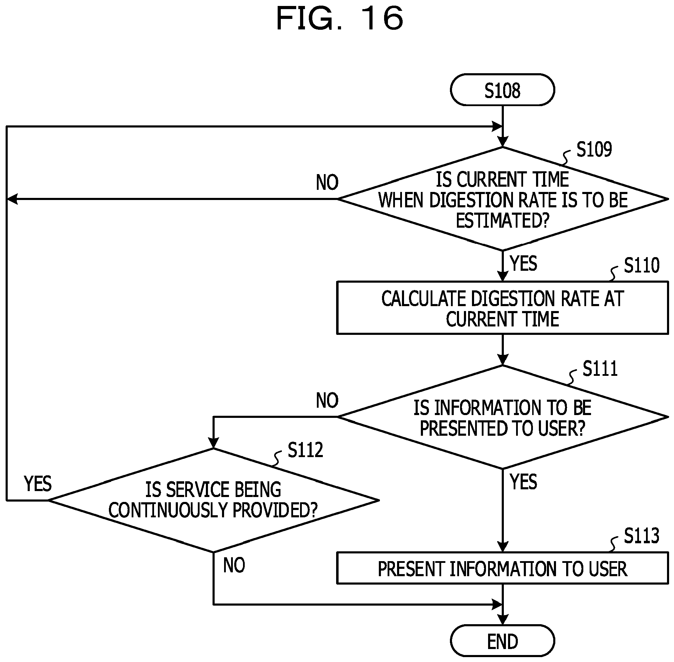

FIG. 14 is a diagram illustrating an example in which the information is stored in the characteristic amount management table 12c in S106. As illustrated in FIG. 16, the characteristic amount calculator 13 causes information of the identified digestion end time 15:45 to be stored in a cell corresponding to the meal ID = 1 and included in the "characteristic amount 4" item. In addition, the characteristic amount calculator 13 causes the value of 200 minutes that is a time period from the identified measurement time of the peak value to the digestion end time to be stored in a cell corresponding to the meal ID = 1 and included in the "characteristic amount 6" item. As described above, the digestion end time may be identified at the end of the predetermined time zone by calculating the primary approximate straight line using the heart rate signal information items measured in the predetermined time zone from the time when the peak value is measured. Thus, the digestion rate may be estimated in real time after the predetermined time zone.

Return to FIG. 4. After the process of S107, the characteristic amount calculator 13 calculates a time period from the meal end time to the measurement time of the peak value (in S108). Specifically, the characteristic amount calculator 13 calculates the time period from the meal end time to the measurement time of the peak value by calculating the difference between the time when the peak value is measured and the meal end time stored in the meal time management table 12b illustrated in FIG. 8. The time when the peak value is measured is 12:25 based on the heart rate management table 12a illustrated in FIG. 6. In addition, based on the meal time management table 12b illustrated in FIG. 8, the meal end time is 12:07. Thus, the time period from the meal end time to the measurement time of the peak value is calculated to be 18 minutes.

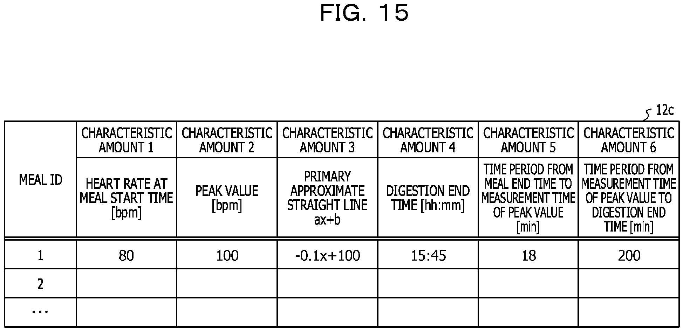

FIG. 15 is a diagram illustrating an example in which the information is stored in the characteristic amount management table 12c in S108. The characteristic amount calculator 13 causes the time period of 18 minutes from the identified meal end time to the measurement time of the peak value to be stored in a cell corresponding to the meal ID = 1 and included in a "characteristic amount 5" item. In the aforementioned manner, the process of S108 is executed. Processes to be executed after S108 are described below.

FIG. 16 is a second flowchart of the example of the digestion rate estimation method to be executed by the information processing device.

After the process of S108, the determiner 15 determines whether or not the current time is the time when the digestion rate is to be estimated (in S109). In the first embodiment, since the digestion rate is calculated at time intervals set in advance, the determiner 15 executes the determination process of S109. The results of calculating the digestion rate are stored in the storage section 12, and the digestion rate estimation management table 12d for management is prepared in the storage section 12.

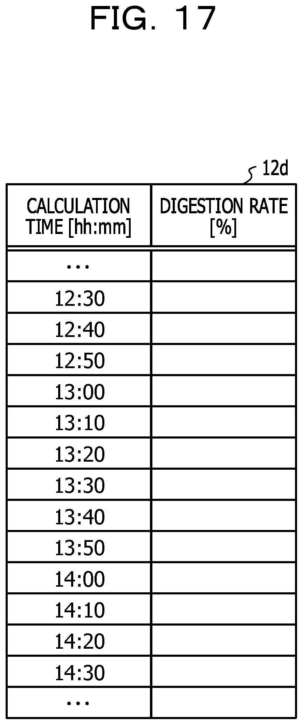

FIG. 17 is a diagram illustrating an example of an initial state of the digestion rate estimation management table 12d. The digestion rate estimation management table 12d includes a digestion rate item and a calculation time item indicating time when the digestion rate is calculated. In the example illustrated in FIG. 17, the digestion rate is calculated at time intervals of 10 minutes, and multiple calculation time points when the digestion rate is calculated are set so that each of intervals between the calculation time points is 10 minutes. Each of the intervals between the set calculation time points may be 1 minute or 1 second.

Return to FIG. 16. In S109, the determiner 15 reads information of the current time and determines whether or not the current time has reached any of the multiple calculation time points set in advance, thereby determining whether or not the current time is the time when the digestion rate is to be estimated. If the determiner 15 determines that the current time is not the time when the digestion rate is to be estimated (No in S109), the process of S109 is executed again. On the other hand, if the determiner 15 determines that the current time is the time when the digestion rate is to be estimated (Yes in S109), the digestion rate estimator 14 calculates the digestion rate at the current time (in S110).

The digestion rate estimator 14 treats the meal end time as the time (digestion start time) when the digestive activity occurs in the stomach, and the digestion rate estimator 14 sets the digestion rate at the meal end time to 0% and the digestion rate at the digestion end time to 100%. Then, the digestion rate estimator 14 calculates the digestion rate at the current time based on time elapsed after the meal end time. Specifically, the digestion rate estimator 14 calculates the digestion rate by calculating the ratio of the length of a time period from the meal end time to the current time with respect to the length of a time period from the meal end time to the digestion end time or a digestion time period.

As illustrated in FIG. 12, the length of the digestion time period may be calculated by calculating the sum of the "characteristic amount 5" of the characteristic amount management table 12c and the "characteristic amount 6" of the characteristic amount management table 12c. Thus, the digestion rate at the current time may be calculated according to the following equation.

$$\text{The digestion rate} = [(\text{the current time} - \text{the meal end time}) / (\text{the digestion time period})] \times 100\% = [(\text{the current time} - \text{the meal end time}) / (\text{the characteristic amount 5} + \text{the characteristic amount 6})] / \times 100\%$$

For example, it is assumed that the current time is 12:30. Since the meal end time is 12:07 based on the meal time management table 12b illustrated in FIG. 8, the difference between the current time and the meal end time is calculated to be 23 minutes. In addition, based on the characteristic amount management table 12c illustrated in FIG. 15, the "characteristic amount 5" is 18 minutes, and the "characteristic amount 6" is 200 minutes. Thus, the aforementioned equation is used to calculate the digestion rate at the current time of 12:30 as $[23 / (18 + 200)] \times 100\% \approx 11\%$. The digestion rate estimator 14 associates the calculated value of 11% of the digestion rate with a calculation time point and causes the calculated value of 11% to be stored in the digestion rate management table 12d.

For example, it is assumed that the current time is 12:30. Since the meal end time is 12:07 based on the meal time management table 12b illustrated in FIG. 8, the difference between the current time and the meal end time is calculated to be 23 minutes. In addition, based on the characteristic amount management table 12c illustrated in FIG. 15, the "characteristic amount 5" is 18 minutes, and the "characteristic amount 6" is 200 minutes. Thus, the aforementioned equation is used to calculate the digestion rate at the current time of 12:30 as $[23 / (18 + 200)] \times 100\% \approx 11\%$. The digestion rate estimator 14 associates the calculated value of 11% of the digestion rate with a calculation time point and causes the calculated value of 11% to be stored in the digestion rate management table 12d.

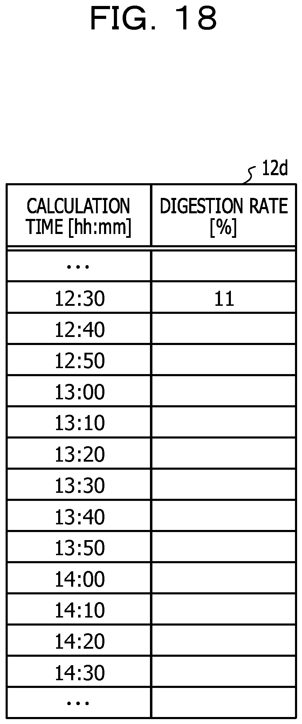

FIG. 18 is a diagram illustrating an example in which the information is stored in the digestion rate management table 12d in S110. As illustrated in FIG. 18, the calculated value of 11% is stored in a cell corresponding to the calculation time point of 12:30 and included in the digestion rate item, for example.

Subsequently, the determiner 15 determines whether or not information on the service is to be presented to the user of the information processing device 10 (in S111). Examples of the service are a service for presenting an eating place such as a recommended restaurant when the user becomes hungry and a service for presenting appropriate food ingestion time to a user who goes on a diet and providing advice to the user. In addition, examples of the service are a service for promoting a user who is worry about a digestion ability to eat food when digestion ends or almost ends and a service for providing advice about appropriate meal time to an elderly user. When the digestion rate reaches a predetermined digestion rate, the determiner 15 determines that the information on the service is to be presented to the user in S111. The predetermined digestion rate varies depending on details of the service, but may be, for example, in a range of 95% to 100%.

If the determiner 15 determines that the information on the service is not to be presented to the user (No in S111), the determiner 15 determines whether or not the service is being continuously provided to the user (in S112). For example, in S112, the determiner 15 determines whether or not the information processing device 10 is continuously using an application related to the service, and the determiner 15 determines, based on the result of the determination, whether or not the service is being continuously provided to the user. If the determiner 15 determines that the service is being continuously provided to the user (Yes in S112), the method returns to S109, and processes of S109 and later are executed. If the determiner 15 determines that the service is not being continuously provided to the user (No in S112), the series of processes is terminated.

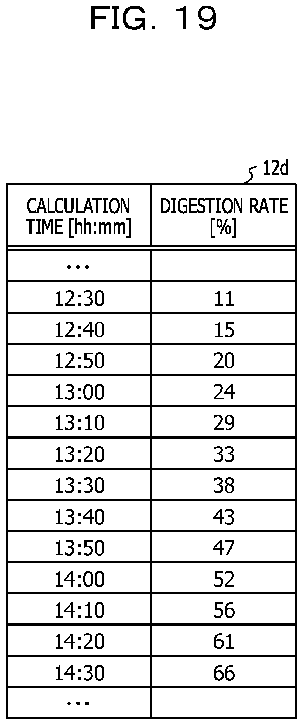

FIG. 19 is a diagram illustrating an example in which information is stored in the digestion rate management table 12d multiple times. As illustrated in FIG. 19, the process of S111 is repeatedly executed until the answer to S111 is positive or until the determiner 15 determines that the information on the service is to be presented to the user. Then, the digestion rate is calculated at time intervals of 10 minutes, and the results of the calculation are gradually accumulated in the digestion rate management table 12d. At 14:20, the digestion rate is 61%. The calculation of the digestion rate and the accumulation of the results of calculating the digestion rate are repeated until the digestion rate reaches the predetermined digestion rate.

If the determiner 15 determines that the information on the service is to be presented to the user (Yes in S111), the information presenter 16 uses the display device 66 to present the information to the user (in S113). If the information to be presented to the user includes information of a store such as an eating place or a facility, the information processing device 10 acquires the information to be presented to the user via a network from an external device such as a server having a database (DB) storing related information. In another method, the DB storing the related information may be stored in the storage section 12 of the information processing device 10, and information to be used may be read from the storage section 12. After the process of S113, the series of processes is terminated.

In the aforementioned manner, the process of estimating the digestion rate is executed by the information processing device 10.

According to the first embodiment, the time when the heart rate after the end of the meal returns to the heart rate at the meal start time is identified as the digestion end time based on the heart rate at the meal start time and the trend in which the heart rate increases and decreases over time after the end of the meal. Then, the digestion rate at the current time is estimated based on the digestion end time and the information acquired by the second receiver 11b and indicating the meal end time. According to this method, since the latest digestion rate may be estimated based on the latest heart rate signal information item among heart rate signal information items sequentially received, the service based on the estimated digestion rate may be provided to the user in real time. For example, if the user receives a service of providing information of a restaurant based on the digestion rate, an operation of searching the restaurant may be omitted.

Modified Example

A modified example of the first embodiment is described below.

In the first embodiment, the peak value measured in the time zone after the end of the meal is identified, and the primary approximate straight line is calculated using the multiple heart rate signal information items measured in the predetermined time zone from the time when the peak value is measured to the time when the set time period ends. However, if the decrease in the heart rate during the predetermined time zone is small, the inclination of the primary approximate straight line is small and the calculated value of the digestion time period may be longer than the actual digestion time period.

In the modified example, if the digestion time period exceeds an upper limit set in advance, the time period set to calculate the primary approximate straight line is changed to a longer time period, the number of sample heart rate signal information items is increased, and the process of calculating the primary approximate straight line is executed again.

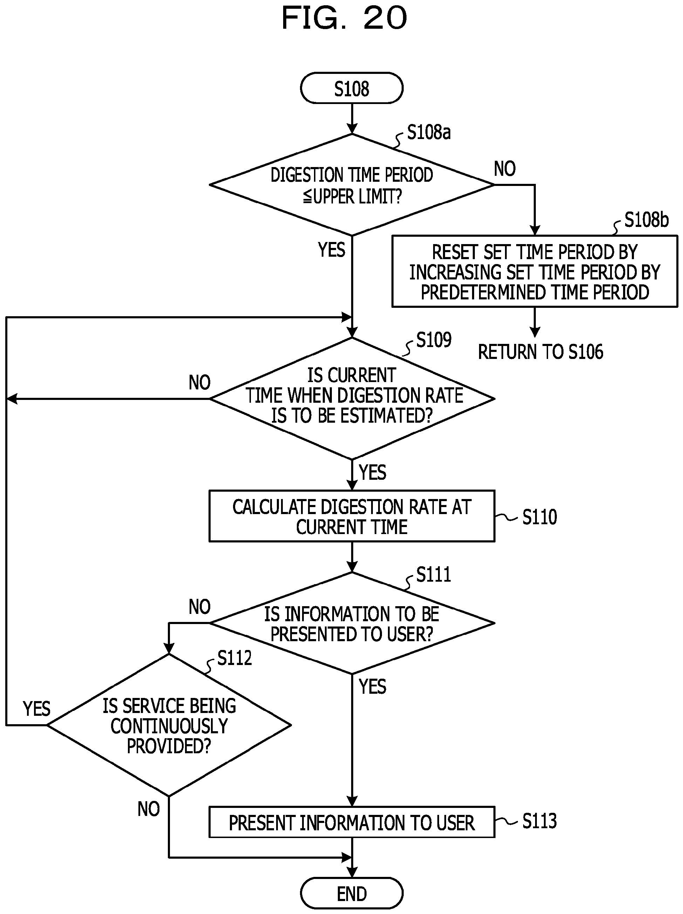

FIG. 20 is a diagram illustrating a modified example of the flowchart illustrated in FIG. 16. Processes that are the same as those included in the process illustrated in FIG. 16 are indicated by the same reference symbols as those illustrated in FIG. 16.

The digestion time period is calculated as the sum of the "characteristic amount 5" and the "characteristic amount 6". In the modified example, after the process of S108, the determiner 15 determines whether or not the calculated digestion time period is equal to or smaller than the upper limit in order to the validity of the calculated digestion time period (in S108a). The upper limit is a value that is hardly expected as a time period during which ingested food remains in the stomach for digestion. The upper limit is, for example, 8 hours. If the calculated digestion time period exceeds the upper limit (No in S108a), the characteristic amount calculator 13 increases, by a predetermined value, the number of sample heart rate signal information items to be used to calculate the primary approximate straight line (in S108b). Specifically, for example, the characteristic amount calculator 13 adds 5 minutes to the set time period that elapses from the time when the peak value is measured. For example, if the initial set value is 20 minutes, the set value is changed to 25 minutes (= 20 + 5). After the process of S108b, the method returns to S106 illustrated in FIG. 4, and the characteristic amount calculator 13 executes the process of calculating the primary approximate straight line again.

On the other hand, if the determiner 15 determines that the calculated digestion time period is equal to or smaller than the upper limit (Yes in S108a), the method proceeds to S109, and processes of S109 and later are executed. The processes of S109 and later are substantially the same as those included in the process illustrated in FIG. 16, and a description thereof is omitted.

In the aforementioned manner, the process of estimating the digestion rate is executed by the information processing device 10.

According to the modified example of the first embodiment, the determiner 15 determines whether or not the calculated digestion time period is equal to or smaller than the upper limit, and if the determiner 15 determines that the digestion time period exceeds the upper limit, the set time period is increased by the predetermined value and is reset. According to this method, since the primary approximate straight line may be repeatedly calculated until a downward trend in the heart rate after the meal appears, the primary approximate straight line may be more appropriately calculated. Thus, the accuracy of the identification of the digestion time period may be improved.

Second Embodiment

Next, a second embodiment is described. In the first embodiment, the information processing device carried by the user who receives the service based on the digestion rate executes the process of estimating the digestion rate. On the other hand, in the second embodiment, an information processing device that is different from an information processing device carried by the user executes a process of estimating the digestion rate instead.

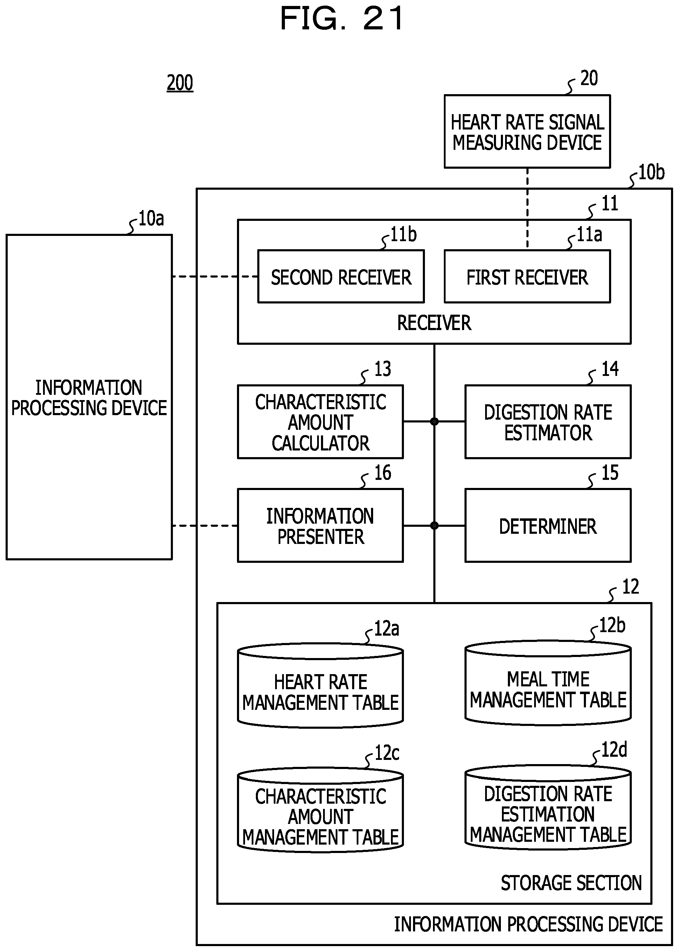

FIG. 21 is a diagram illustrating an example of an information processing system 200 according to the second embodiment. Functional blocks that are the same as the functional blocks illustrated in FIG. 1 are indicated by the same reference symbols as those illustrated in FIG. 1, and a description thereof is omitted. As illustrated in FIG. 21, the information processing system 200 includes an information processing device 10a, an information processing device 10b, and the heart rate measuring device 20. Broken lines illustrated in FIG. 2 indicate wireless communication.

The information processing device 10a is a computer carried by the user, like the information processing device 10 illustrated in FIG. 1. The information processing device 10a is, for example, a smartphone, a mobile phone, a tablet terminal, a laptop computer, a wearable computer, or the like.

The information processing device 10b is a computer that estimates the digestion rate of the user carrying the information processing device 10a. The information processing device 10b is installed at a location away from the user. The information processing device 10b is, for example, a server, a desktop computer, or the like. The information processing device 10b includes the receiver 11, the storage section 12, the characteristic amount calculator 13, the digestion rate estimator 14, the determiner 15, and the information presenter 16, like the information processing device 10 illustrated in FIG. 1.

The information processing system 200 may be achieved as a wearable computer including the information processing device 10b and the heart rate signal measuring device 20.

Next, the flow of a process, to be executed by the information processing device 10b, of estimating the digestion rate and presenting information to the user based on the result of the estimation is described.

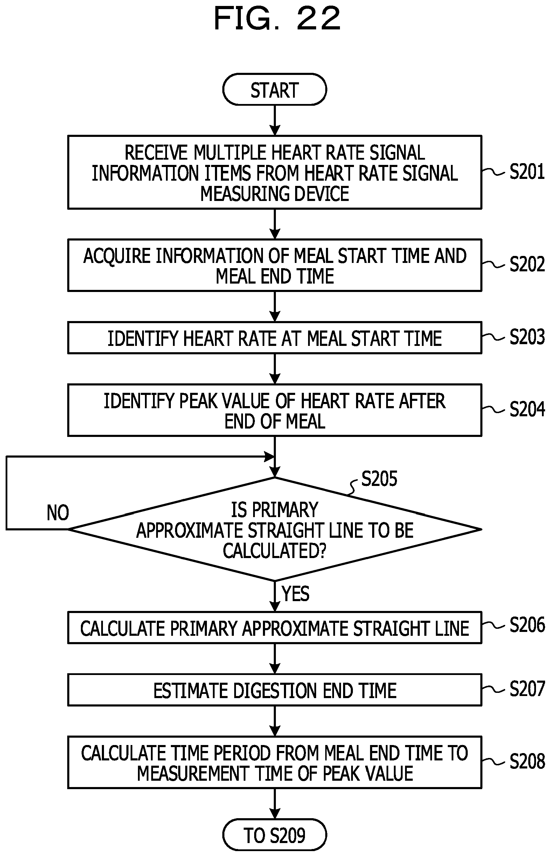

FIG. 22 is a flowchart of an example of a digestion rate estimation method to be executed by the information processing device 10b according to the second embodiment.

First, the receiver 11 acquires heart rate signal information items from the heart rate signal measuring device 20 (in S201). The process of S201 is substantially the same as the process of S101 illustrated in FIG. 1 and described in the first embodiment, and a description thereof is omitted.

Subsequently, the receiver 11 acquires information of meal start time and meal end time from the information processing device 10a (in S202). Specifically, the user who carries the information processing device 10a uses the input device 65 included in the information processing device 10a to enter the information of the meal start time and the meal end time. Then, the information processing device 10a transmits the entered information to the receiver 11 of the information processing device 10b. Thus, the information processing device 10b acquires the information of the meal start time and the meal end time. Alternatively, an acceleration sensor worn by the user or a body temperature microphone worn by the user is used to identify the meal start time and the meal end time by detecting an operation of the user and transmit the identified information to the receiver 11 of the information processing device 10b. Thus, the information processing device 10b acquires the information of the meal start time and the meal end time.

Processes of S203 to S208 are substantially the same as the processes of S103 to S108 illustrated in FIG. 4 and described in the first embodiment, and a description thereof is omitted.

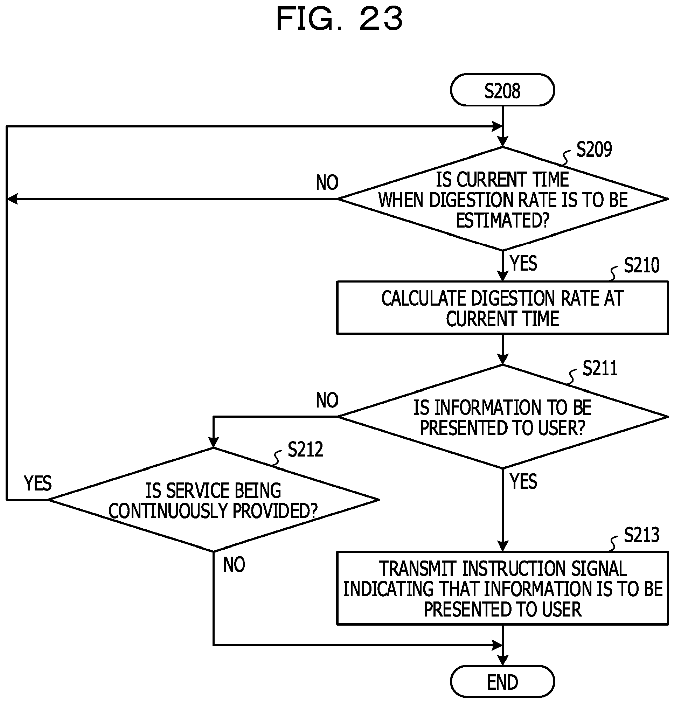

FIG. 23 is a second flowchart of the example of the digestion rate estimation method to be executed by the information processing device 10b according to the second embodiment.

Processes of S209 to S211 are substantially the same as the process of S109 to S111 illustrated in FIG. 16 and described in the first embodiment, and a description thereof is omitted. If the determiner 15 determines that the information is not to be presented to the user (No in S211), the determiner 15 determines whether or not the service is being continuously provided to the user (in S212). For example, in S212, the information processing device 10b determines whether or not the information processing device 10b has received a signal indicating the stop of the use of the application related to the service and executed by the information processing device 10a, thereby determining whether or not the service is being continuously provided to the user. If the determiner 15 determines that the service is being continuously provided to the user (Yes in S212), the method returns to S209, and processes of S209 and later are executed again. If the determiner 15 determines that the service is not being continuously provided to the user (No in S212), the series of processes is terminated.

On the other hand, if the determiner 15 determines that the information is to be presented to the user (Yes in S211), the information presenter 16 transmits, to the information processing device 10a, an instruction signal indicating that the information is to be presented to the user (in S213). If the information to be presented to the user includes information of a store such as an eating place or a facility, the information processing device 10b acquires the information to be presented to the user via a network from an external device such as a server having a database (DB) storing related information. The information processing device 10b may transmit the acquired information and the instruction signal. In another method, the DB storing the related information may be stored in the storage section 12 of the information processing device 10b, and information to be used may be read from the storage section 12. The information processing device 10b may include the DB and may not communicate with the external device in order to acquire the service information. After the process of S213, the series of processes is terminated.

According to the second embodiment, the process of estimating the digestion rate is executed by the information processing device 10b installed at the location away from the user, instead of the information processing device 10a carried by the user, and the various tables to be used to estimate the digestion rate are stored in the information processing device 10b installed at the location away from the user, instead of the information processing device 10a carried by the user. Thus, the load of a processor of the information processing device carried by the user may be reduced, and a storage region that is to be used and is included in a memory of the information processing device carried by the user may be reduced.

Third Embodiment

Next, a third embodiment is described. In the first embodiment, the various tables to be used for the process of estimating the digestion rate are stored in the information processing device 10 carried by the user. In the third embodiment, the various tables are not stored in an information processing device 10 carried by the user and are stored in an external storage device.

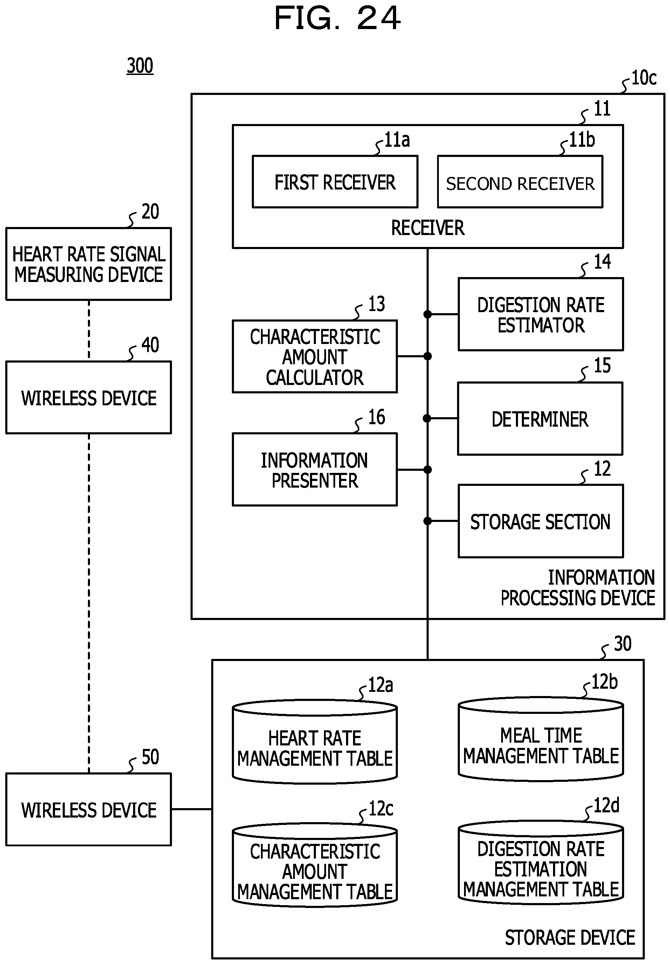

FIG. 24 is a diagram illustrating an example of an information processing system 300 according to the third embodiment. Functional blocks that are the same as the functional blocks illustrated in FIG. 1 are indicated by the same reference symbols as those of the functional blocks illustrated in FIG. 1, and a description thereof is omitted. As illustrated in FIG. 24, the information processing system 300 includes an information processing device 10c, the heart rate signal measuring device 20, a memory device 30, a wireless device 40, and a wireless device 50. Broken lines illustrated in FIG. 24 indicate wireless communication.

The information processing device 10c is a computer carried by the user, like the information processing device 10 according to the first embodiment. Specifically, the information processing device 10c estimates the digestion rate and provides the service to the user based on the result of the estimation, like the information processing device 10. The information processing device 10c is, for example, a smartphone, a mobile phone, a tablet terminal, a laptop computer, a wearable computer, or the like. The information processing device 10c may be achieved as a wearable computer including the heart rate signal measuring device 20. The information processing device 10c includes the receiver 11, the storage section 12, the characteristic amount calculator 13, the digestion rate estimator 14, the determiner 15, and the information presenter 16, like the information processing device 10 illustrated in FIG. 1. In the third embodiment, the storage section 12 functions as a nonvolatile memory for storing the program (digestion rate estimation program or the like) and a cache memory for temporarily storing data to be used to execute the program.

The memory device 30 is used as a DB for storing various types of information to be used for processes to be executed by the information processing device 10c and is achieved by a storage device or a server. The information processing device 10c may execute the processes by reading data from the memory device 30 and storing data in the memory device 30. The information processing device 10c may use the memory device 30 as a cloud server by accumulating various types of information in the memory device 30.

In addition, the wireless device 40 is connected to the heart rate signal measuring device 20, while the wireless device 50 is connected to the memory device 30. The wireless devices 40 and 50 are achieved by wireless interfaces, for example. The wireless device 40 may be included in the heart rate signal measuring device 20, while the wireless device 50 may be included in the memory device 30.

The heart rate signal measuring device 20 transmits information of the heart rate measured at predetermined time intervals to the memory device 30 via the wireless communications 40 and 50 by wireless communication in each of measurement cycles or at predetermined time when batch processing is executed. The information received by the memory device 30 and indicating the heart rate is accumulated in the heart rate management table 12a over time.

A process, to be executed by the information processing device 10c, of estimating the digestion rate is started from the process of S102 illustrated in FIG. 4. The information processing device 10c executes the processes of S103 and later while reading information to be used for the processes from the various tables stored in the memory device 30 or referencing the various tables. Details of the processes are substantially the same as those described in the first embodiment, and a description thereof is omitted.

According to the third embodiment, the various tables to be used to estimate the digestion rate are not stored in the information processing device carried by the user and are stored in the external memory device. Thus, a storage region that is to be used and is included in a memory of the information processing device carried by the user may be reduced.

Fourth Embodiment

Next, a fourth embodiment is described. In the first to third embodiments, the processes of estimating the digestion rate using the heart rate profile corresponding to the time period from time before the meal to time after the meal are executed. In the fourth embodiment, a change rate and digestion rate correspondence table is generated as a model indicating relationships between the rate of change of the heart rate and the digestion rate and learned, and the digestion rate is estimated based on the model.

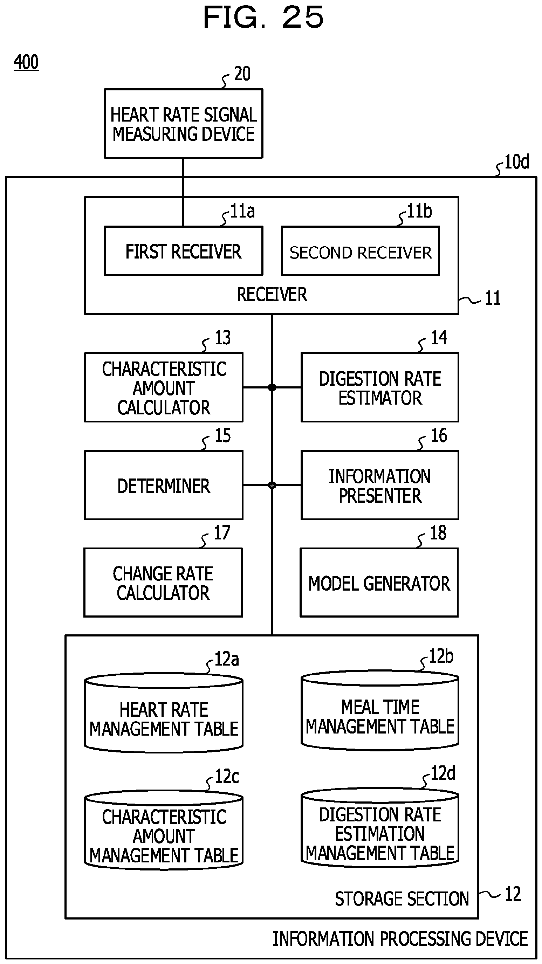

FIG. 25 is a diagram illustrating an example of an information processing system 400 according to the fourth embodiment. Functional blocks that are the same as the functional blocks illustrated in FIG. 1 are indicated by the same reference symbols as those of the functional blocks illustrated in FIG. 1, and a description thereof is omitted. As illustrated in FIG. 25, the information processing system 400 includes an information processing device 10d and the heart rate signal measuring device 20.

The information processing device 10d is a computer carried by the user. The information processing device 10d is, for example, a smartphone, a mobile phone, a tablet terminal, a laptop computer, a wearable computer, or the like. The information processing device 10d includes the functional blocks included in the information processing device 10 illustrated in FIG. 1 and includes a change rate calculator 17 and a model generator 18. The change rate calculator 17 and the model generator 18 are achieved by, for example, the CPU 61 illustrated in FIG. 2.

The change rate calculator 17 calculates the rate of change of the heart rate based on the heart rate profile. The rate of change of the heart rate is defined as the amount of a change in the heart rate per unit of time.

The model generator 18 uses information of the rate, calculated by the change rate calculator 17, of change of the heart rate to generate the change rate and digestion rate correspondence table. The change rate and digestion rate correspondence table is referenced upon the estimation of the digestion rate.

The information processing system 400 may be achieved as a wearable computer including the information processing device 10d and the heart rate signal measuring device 20.

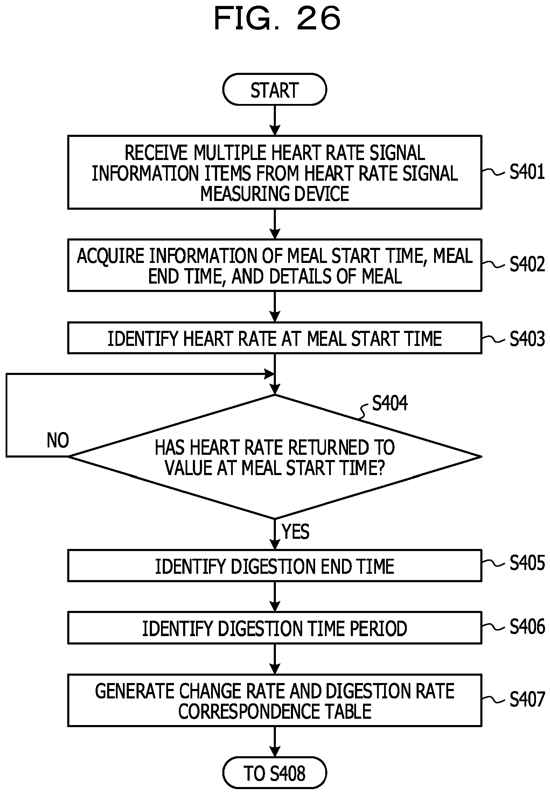

FIG. 26 is a first flowchart of an example of a digestion rate estimation method to be executed by the information processing device 10d according to the fourth embodiment. FIG. 26 illustrates a process of generating the change rate and digestion rate correspondence table.

First, the receiver 11 acquires heart rate signal information items from the heart rate signal measuring device 20 (in S401). The process of S401 is substantially the same as the process of S101 illustrated in FIG. 4 and described in the first embodiment.

Subsequently, the receiver 11 acquires information of the meal start time, the meal end time, and details of the meal (in S402). A process of acquiring the meal start time and the meal end time is substantially the same as the process of S102 illustrated in FIG. 4, but the information of the meal start time, the meal end time, and the details of the meal is acquired in the fourth embodiment. This is due to the fact that the heart rate profile and a profile (described later) of the rate of change of the heart rate vary depending on the details of the meal or the speed at which the user eats food. Specifically, in the fourth embodiment, the digestion rate may be estimated with high accuracy by generating a change rate and digestion rate correspondence table for each meal, regardless of details of the meal or the speed at which the user eats food. The receiver 11 causes the acquired information of the meal start time, the meal end time, and the details of the meal to be stored in the storage section 12.

After the process of S402, the characteristic amount calculator 13 identifies the heart rate at the meal start time (in S403). The process of S403 is substantially the same as the process of S103 illustrated in FIG. 4.

After the process of S403, the determiner 15 determines whether or not the heart rate has returned to the value at the meal start time (in S404). If the determiner 15 determines that the heart rate has not returned to the value at the meal start time (No in S404), the determiner 15 determines that the digestive activity does not end, and the process of S404 is executed again. On the other hand, if the determiner 15 determines that the heart rate has returned to the value at the meal start time (Yes in S404), the characteristic amount calculator 13 identifies the digestion end time based on the heart rate profile obtained based on the heart rate signal information items accumulated in the storage section 12 (in S405). Specifically, the characteristic amount calculator 13 references the profile of the change in the heart rate over time and identifies, as the digestion end time, the time when the heart rate has returned to the value at the meal start time.

Subsequently, the characteristic amount calculator 13 identifies the digestion time period by calculating the difference between the meal start time acquired in S402 and the digestion end time identified in S405 (in S406). For example, if the heart rate profile illustrated in FIG. 3 is acquired, the digestion time period is identified as 240 minutes.

Subsequently, the model generator 18 generates a change rate and digestion rate correspondence table corresponding to the information acquired in S402 and indicating the details of the meal (in S407). Specifically, it is assumed that the digestion rate at the meal start time is 0%, the digestion rate at the meal end time is 100%, and the digestion rate is proportional to time. Then, the change rate calculator 17 uses the heart rate signal information items received in S401 to calculate the rate of change of the heart rate for the digestion rate in a range of 0% to 100% in units of 10%. For example, if the digestion time period is 240 minutes, a time period for a change in the digestion rate from 0% to 10% is 24 minutes. If the heart rate when the digestion rate is 0% is 60, and the heart rate when the digestion rate is 10% is 67, the rate of change of the heart rate for the digestion rate of 10% is calculated to be 0.29 bpm ($\approx$ (67 - 60) / 24). Then, the model generator 18 generates the change rate and digestion rate correspondence table using the rate, calculated by the change rate calculator 17 for each digestion rate, of change of the heart rate.

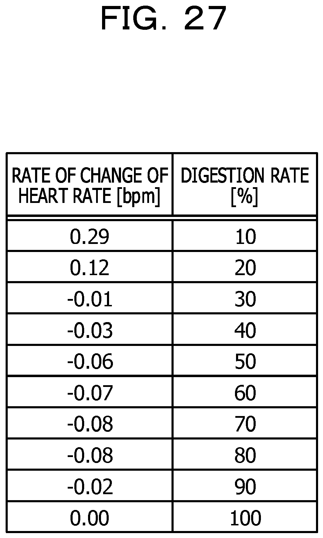

FIG. 27 is a diagram illustrating an example of the change rate and digestion rate correspondence table. FIG. 27 illustrates an example of the case where the heart rate profile illustrated in FIG. 3 is acquired. The change rate and digestion rate correspondence table includes an item for rates of change of the heart rate and an item for digestion rates. When the heart rate increases, the rate of change of the heart rate is a positive value. When the heart rate decreases, the rate of change of the heart rate is a negative value. When the rate of change of the heart rate changes from a negative value to 0, the digestion rate is 100%.

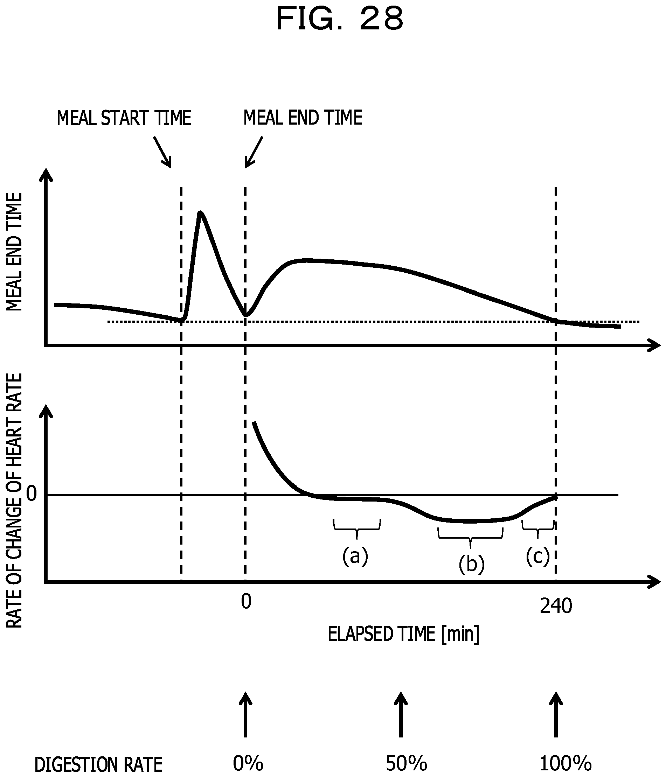

FIG. 28 is a diagram illustrating the profile of the rate of change of the heart rate in order to describe the change rate and digestion rate correspondence table illustrated in FIG. 27. A profile illustrated on the upper side of FIG. 28 indicates the same heart rate profile as that illustrated in FIG. 3. The profile illustrated on the lower side of FIG. 28 indicates the profile, corresponding to the profile illustrated on the upper side of FIG. 28, of the rate of change of the heart rate. As illustrated on the lower side of FIG. 28, at the time when the digestion rate is 10% immediately after the end of the meal, the rate of change of the heart rate is 0.29 and a positive value. However, when the user ends the meal and digestion starts, the rate of change of the heart rate gradually decreases. Then, the rate of change of the heart rate changes from a positive value to a negative value before the digestion rate becomes 30%. When the digestion rate is 30%, the rate of change of the heart rate is -0.01. After that, the rate of change of the heart rate gradually decreases until the digestion rate becomes close to 50%. Then, when the digestion rate exceeds 80%, the rate of change of the heart rate increases again. Then, when the digestion rate is 100%, the rate of change of the heart rate is 0%. The change rate and digestion rate correspondence table illustrated in FIG. 27 is information obtained by modeling the profile of the rate of change of the heart rate. The information processing system 400 may generate and learn multiple change rate and digestion rate correspondence tables for meals.

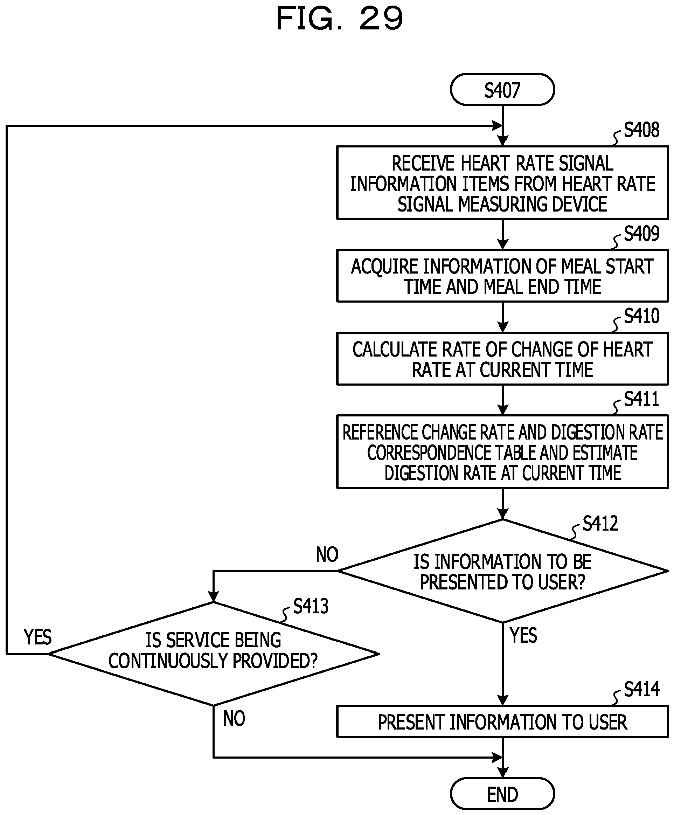

Return to FIG. 26. After the model generator 18 generates the change rate and digestion rate correspondence table in S407, the model generator 18 causes the generated table to be stored in the storage section 12. After that, the method returns to S408 illustrated in FIG. 29.

FIG. 29 is a second flowchart of the example of the digestion rate estimation method to be executed by the information processing device 10d according to the fourth embodiment. FIG. 29 illustrates a process of estimating the digestion rate using the generated change rate and digestion rate correspondence table and providing the service to the user based on the estimated digestion rate.

In S408, the receiver 11 receives, from the heart rate signal measuring device 20, heart rate signal information items including information of the heart rate at the current time.

Subsequently, the receiver 11 acquires information of the meal start time and the meal end time (in S409). The process of S409 is substantially the same as the process of S102 described in the first embodiment.

Subsequently, the change rate calculator 17 calculates the rate of change of the heart rate at the current time by using the heart rate at the current time and a heart rate signal information item received before the current time (in S410). The heart rate signal information item received before the current time may be the single heart rate signal information item or may be multiple heart rate signal information items received in a past predetermined time zone (of 10 minutes or the like).

Subsequently, after the rate of change of the heart rate at the current time is calculated, the digestion rate estimator 14 estimates the digestion rate at the current time by referencing the change rate and digestion rate correspondence table generated in S407 (in S411). A method for estimating the digestion rate is described below.

Since the rate of change of the heart rate is an almost fixed value during a time zone indicated by (b) in FIG. 28, the same value as the rate of change of the heart rate at the current time may be associated with multiple digestion rates continuously registered in the change rate and digestion rate correspondence table. In this case, the multiple digestion rates associated with the rate of change of the heart rate at the current time are extracted. During the time zone indicated by (b), the digestion rate is in a range of 70% to 80%, and the information is not presented to the user. Thus, as long as the service based on the digestion rate is provided, the digestion rate at the current time is identified to be a value in the range of 70% to 80%, and a determination process of S412 may be executed after S411.

On the other hand, the same value as the rate of change of the heart rate at the current time may be associated with multiple digestion rates discontinuously registered in the change rate and digestion rate correspondence table. For example, a certain rate of change of the heart rate in a time zone indicated by (a) in FIG. 28 is equal to a certain rate of change of the heart rate in a time zone indicated by (c) in FIG. 28. Even if the rate of change of the heart rate at the current time is searched from the change rate and digestion rate correspondence table, it is difficult to estimate whether the digestion rate at the current time is the digestion rate in the time zone indicated by (a) or the digestion rate in the time zone indicated by (c).

In this case, the digestion rate estimator 14 may reference history records of the rate, calculated before the current time, of change of the heart rate and estimate whether the digestion rate is equal to the value in the time zone indicated by (a) or the value in the time zone indicated by (c). For example, if the rate of change of the heart rate at the current time is lower than the rate, calculated in the process of S410 immediately before the current time, of change of the heart rate, the digestion rate estimator 14 may estimate that the digestion rate at the current time is equal to the value in the time zone indicated by (a). On the other hand, if the rate of change of the heart rate at the current time is higher than the rate, calculated in the process of S410 immediately before the current time, of change of the heart rate, the digestion rate estimator 14 may estimate that the digestion rate at the current time is equal to the value in the time zone indicated by (c).

Alternatively, if the details of the meal are known in advance, the digestion rate estimator 14 may estimate whether the digestion rate at the current time is equal to the value in the time zone indicated by (a) or the value in the time zone indicated by (c), based on an elapsed time period from the meal end time acquired in S409 to the current time. For example, if it is known that ingested food corresponds to a digestion time period of approximately 4 hours, and the elapsed time period from the meal end time to the current time is 2 hours or less, the digestion rate estimator 14 may estimate that the digestion rate at the current time is equal to the value in the time zone indicated by (a). On the other hand, if the elapsed time period from the meal end time to the current time exceeds 2 hours, the digestion rate estimator 14 may estimate that the digestion rate at the current time is equal to the value in the time zone indicated by (c).

In the aforementioned manner, the process of S411 is executed.

Subsequently, the determiner 15 determines, based on the estimated value of the digestion rate, whether or not the information on the service is to be presented to the user of the information processing device 10d (in S412).

Since the process of S412 is substantially the same as the process of S111 illustrated in FIG. 16 and described in the first embodiment, the determiner 15 compares the estimated value of the digestion rate with a predetermined digestion rate threshold, thereby determining whether or not the information on the service is to be presented. If multiple candidates of the digestion rate are extracted, and a candidate among the candidates of the digestion rate has reached a predetermined digestion rate, the determiner 15 determines that the information on the service is to be presented.

Processes of S413 and S414 illustrated in FIG. 29 are substantially the same as the processes S112 and S113 illustrated in FIG. 16 and described in the first embodiment, and a description thereof is omitted. In the aforementioned manner, the process according to the fourth embodiment is executed.

According to the fourth embodiment, the entire heart rate profile measured during a time period from the end of the meal to the end of the digestive activity is acquired in advance, and the change rate and digestion rate correspondence table indicating association relationships between the rate of change of the heart rate and the digestion rate is generated based on the heart rate profile. Then, the digestion rate at the current time is estimated using the change rate and digestion rate correspondence table. According to this method, since an error of the digestion time period is reduced by using the heart rate profile acquired in advance, the accuracy of estimating the digestion rate may be improved.

Although the embodiments are described above, the techniques disclosed herein are not limited to the embodiments and may be variously modified and changed. In each of the embodiments, the digestion rate is estimated, but the degree of hunger, a satiety level, or the like may be estimated instead of the digestion rate.

In the first embodiment, the primary approximate straight line is calculated using the multiple heart rate signal information items measured in the time period from the time when the peak value is measured to the time after the elapse of the set time period. For example, if the current time is after the elapse of the set time period, the primary approximate straight line may be calculated using multiple heart rate signal information items measured in a time period from the time when the peak value is measured to the current time. According to this method, as a time period after the elapse of the set time period is longer, the number of heart rate signal information items to be used to calculate the primary approximate straight line is larger. Thus, the accuracy of calculating the primary approximate straight line may be improved and the digestion end time may be more accurately identified.

In the first to third embodiments, the digestion end time is identified using the primary approximate straight line, but may be identified using a high-order curve such as a quadratic curve. For example, if the digestion rate is estimated to be in a range of 50% to 100%, heart rate signal information items are sufficiently accumulated, and the digestion end time may be identified using the high-order curve with high accuracy.

In the fourth embodiment, the change rate and digestion rate correspondence table is generated and learned, and the digestion rate is estimated based on the model. The digestion rate, however, may be estimated by learning the heart rate profile measured during the time period from the end of the meal to the end of the digestive activity in advance and referencing the heart rate profile.

The aforementioned information processing devices, the computer program for causing the computers to execute the digestion rate estimation methods, and a non-transitory computer-readable storage medium storing the program are included in claims. The non-transitory computer-readable storage medium is, for example, a memory card such as an SD memory card. The aforementioned computer program may not be stored in the storage medium and may be transmitted via a telecommunication line, a wireless or wired communication line, a network represented by the Internet, or the like.

## Claims

1. An information processing device (10) configured to estimate a digestion rate of food ingested by a user, the information processing device (10) comprising:

   a first receiver (11a) configured to receive, from a heart rate signal measuring device (20) carried by the user, a plurality of heart rate signal information items measured at predetermined time intervals;
   a second receiver (11b) configured to receive information of meal start time and meal end time of the user;
   a characteristic amount calculator (13) configured to:

   specify a heart rate of the user at the meal start time from the plurality of heart rate signal information items,
   calculate, by using the plurality of heart rate signal information items, a primary approximate straight line obtained by approximating a profile indicating decrease of a heart rate of the user included in a trend of the heart rate after an end of a meal, the trend indicating that the heart rate increases and subsequently decreases, and
   estimate a time point indicating the same value as the heart rate at the meal start time on the calculated primary approximate straight line as the digestion end time indicating a time when the digestion of the food ends; and

a digestion rate estimator (14) configured to calculate the digestion rate by calculating a ratio of a length of a time period from the meal end time to a current time with respect to a length of a time period from the meal end time to the digestion end time; and
an information presenter (16) configured to present information on a service according to the calculated digestion rate to the user.

2. The information processing device (10) according to claim 1, further comprising:

a determiner (15) configured to determine whether or not the digestion rate is equal to or higher than a threshold, wherein the information presenter (16) is configured to present the information on a service to the user when the determiner (15) determines that the digestion rate is equal to or higher than the threshold.

3. The information processing device (10) according to claim 1, wherein the characteristic amount calculator (13) is configured to:

identify the heart rate at the meal start time and a maximum value of the heart rate measured after the end of the meal by referencing the plurality of heart rate signal information items;
use multiple heart rate signal information items that are among the plurality of heart rate signal information items and are measured after a time when the maximum value is measured, thereby calculating a primary approximate straight line obtained by approximating a change in the heart rate measured after the time when the maximum value is measured; and
use the primary approximate straight line to identify, as the digestion end time, a time when the heart rate returns to a value at the meal start time after becoming the maximum value.

4. The information processing device (10) according to claim 3, wherein the characteristic amount calculator (13) is configured to:

set a time period indicating time elapsed after the maximum value is measured; and
calculate the primary approximate straight line using multiple heart rate signal information items measured in a time period from the time when the maximum value is measured to time after an elapse of the set time period.

5. The information processing device (10) according to claim 1, wherein the digestion rate estimator (14) is configured to:

calculate a digestion time period by calculating a difference between the digestion end time and digestion start time that is treated as the meal end time; and
estimate the digestion rate by calculating a ratio of an elapsed time period from the meal end time to the current time with respect to the digestion time period.

6. The information processing device (10) according to claim 2,
wherein the determiner (15) is configured to determine whether or not all multiple heart rate signal information items measured in a time zone from the time when the maximum value is measured to the time after the elapse of the set time period have been acquired, and
wherein the characteristic amount calculator (13) is configured to calculate the primary approximate straight line when the determiner determines that all the multiple heart rate signal information items measured in the time zone have been acquired.

7. The information processing device (10) according to claim 2, wherein the determiner (15) is configured to:

determine whether or not the calculated digestion time period is equal to or smaller than an upper limit, and
reset the set time period by increasing the set time period by a predetermined time period when the determiner determines that the digestion time period exceeds the upper limit.

8. The information processing device (10) according to claim 1, further comprising:

a change rate calculator (17) configured to calculate a rate of change of the heart rate per unit of time based on multiple heart rate signal information items, received by the first receiver (11a), measured in a time period from the end of the meal, to the measurement of the maximum value, to a time when the heart rate returns to the value at the meal start time; and

a model generator (18) configured to generate a correspondence table indicating association relationships between the rate of change of the heart rate and the digestion rate,

wherein the change rate calculator (17) is configured to calculate the rate of change of the heart rate at the current time based on a heart rate signal information item measured at a current time and one or more heart rate signal information items measured before the current time, and

wherein the digestion rate estimator (14) is configured to estimate the digestion rate at the current time based on the rate of change of the heart rate at the current time by referencing the correspondence table.

9. A digestion rate estimation method executed by an information processing device configured to estimate a digestion rate of food ingested by a user carrying a heart rate signal measuring device, the digestion rate estimation method comprising:

receiving, from the heart rate signal measuring device, a plurality of heart rate signal information items measured at predetermined time intervals and receiving information of meal start time and meal end time of the user from a device different from the heart rate signal measuring device;

specifying a heart rate of the user at the meal start time from the plurality of heart rate signal information items;

calculating, by using the plurality of heart rate signal information items, a primary approximate straight line obtained by approximating a profile indicating decrease of a heart rate of the user included in a trend of the heart rate after an end of a meal, the trend indicating that the heart rate increases and subsequently decreases;

estimating a time point indicating the same value as the heart rate at the meal start time on the calculated primary approximate straight line as the digestion end time indicating a time when the digestion of the food ends;

calculating the digestion rate by calculating a ratio of a length of a time period from the meal end time to a current time with respect to a length of a time period from the meal end time to the digestion end time; and

presenting information on a service according to the calculated digestion rate to the user.

10. An information processing system configured to estimate a digestion rate of food ingested by a user, the information processing system comprising:

a heart rate signal measuring device (20); and

an information processing device (10) according to any one of claims 1 to 8 configured to communicate with and coupled to the heart rate signal measuring device (20).

11. A digestion rate estimation program which, when executed by an information processing device (10) configured to estimate a digestion rate of a user having a heart rate signal measuring device (20) and a mobile terminal device, causes the information processing device (10) to execute a process according to claim 9.

**Patentansprüche**

1. Informationsverarbeitungsvorrichtung (10), die konfiguriert ist, eine Verdauungsrate von Nahrung, die von einem Benutzer aufgenommen wurde, zu schätzen, wobei die Informationsverarbeitungsvorrichtung (10) umfasst:

einen ersten Empfänger (11a), der konfiguriert ist, von einer von dem Benutzer getragenen Herzfrequenzsignalmessvorrichtung (20) eine Vielzahl von Herzfrequenzsignal-Informationselementen zu empfangen, die in vorbestimmten Zeitintervallen gemessen werden;

einen zweiten Empfänger (11b), der konfiguriert ist, Informationen über eine Startzeit einer Mahlzeit und eine Endzeit einer Mahlzeit des Benutzers zu empfangen;

einen Kennwertrechner (13), der konfiguriert ist zum:

Spezifizieren einer Herzfrequenz des Benutzers zu der Startzeit der Mahlzeit aus der Vielzahl von Herzfrequenzsignal-Informationselementen, Berechnen, unter Verwendung der Vielzahl von Herzfrequenzsignal-Informationselementen, einer primären angenäherten Geraden, die durch Annäherung eines Profils erhalten wird, das die Abnahme einer Herzfrequenz des Benutzers anzeigt, die in einem Trend der Herzfrequenz nach einem Ende einer Mahlzeit enthalten ist, wobei der Trend angibt, dass die Herzfrequenz zunimmt und anschließend abnimmt, und

Schätzen eines Zeitpunkts, der auf der berechneten primären angenäherten Geraden den gleichen Wert wie die Herzfrequenz zur Startzeit der Mahlzeit angibt, als die Endzeit der Verdauung, die eine Zeit angibt, zu der die Verdauung der Nahrung endet; und

einen Verdauungsratenschätzer (14), der konfiguriert ist, die Verdauungsrate durch Berechnen eines Verhältnisses einer Länge einer Zeitperiode von der Endzeit der Mahlzeit zu einer aktuellen Zeit in Bezug auf eine Länge einer Zeitperiode von der Endzeit der Mahlzeit bis zu der Endzeit der Verdauung zu berechnen; und

einen Informationspräsentator (16), der konfiguriert ist, dem Benutzer Informationen als ein Dienst gemäß der berechneten Verdauungsrate zu präsentieren.

2. Informationsverarbeitungsvorrichtung (10) nach Anspruch 1, ferner umfassend:

einen Bestimmer (15), der konfiguriert ist, zu bestimmen, ob die Verdauungsrate gleich oder höher als ein Schwellenwert ist oder nicht;

wobei der Informationspräsentator (16) konfiguriert ist, dem Benutzer die Informationen als ein Dienst zu präsentieren, wenn der Bestimmer (15) bestimmt, dass die Verdauungsrate gleich oder höher als der Schwellenwert ist.

3. Informationsverarbeitungsvorrichtung (10) nach Anspruch 1, wobei der Kennwertrechner (13) konfiguriert ist zum:

Identifizieren der Herzfrequenz zu der Startzeit der Mahlzeit und eines Maximalwerts der Herzfrequenz, gemessen nach dem Ende der Mahlzeit, durch Referenzieren der Vielzahl von Herzfrequenzsignal-Informationselementen;

Verwenden mehrerer Herzfrequenzsignal-Informationselemente, die sich unter der Vielzahl von Herzfrequenzsignal-Informationselementen befinden und

nach einer Zeit gemessen werden, zu der der Maximalwert gemessen wird, wodurch eine primäre angenäherte Gerade berechnet wird, die durch Annäherung einer Änderung in der Herzfrequenz erhalten wird, die nach der Zeit gemessen wird, zu der der Maximalwert gemessen wird; und

Verwenden der primären angenäherten Geraden zum Identifizieren, als Endzeit der Verdauung, einer Zeit, zu der die Herzfrequenz zu einem Wert der Startzeit der Mahlzeit zurückkehrt, nachdem sie den Maximalwert angenommen hat.

4. Informationsverarbeitungsvorrichtung (10) nach Anspruch 3, wobei der Kennwertrechner (13) konfiguriert ist zum:

Einstellen einer Zeitperiode, die eine nach der Messung des Maximalwerts verstrichene Zeit angibt; und

Berechnen der primären angenäherten Gerade unter Verwendung mehrerer Herzfrequenzsignal-Informationselemente, die in einer Zeitperiode von der Zeit, zu der der Maximalwert gemessen wird, bis zu einer Zeit nach Ablauf der eingestellten Zeitperiode gemessen werden.

5. Informationsverarbeitungsvorrichtung (10) nach Anspruch 1, wobei der Verdauungsratenschätzer (14) konfiguriert ist zum:

Berechnen einer Verdauungszeitperiode durch Berechnen einer Differenz zwischen der Endzeit der Verdauung und

der Startzeit der Verdauung, die als Endzeit der Mahlzeit behandelt wird; und

Schätzen der Verdauungsrate durch Berechnen eines Verhältnis einer verstrichenen Zeitperiode von der Endzeit der Mahlzeit zu der aktuellen Zeit in Bezug auf die Verdauungszeitperiode.

6. Informationsverarbeitungsvorrichtung (10) nach Anspruch 2,
wobei der Bestimmer (15) konfiguriert ist, zu bestimmen, ob alle mehreren in einer Zeitzone gemessenen Herzfrequenzsignal-Informationselemente von der Zeit, zu der der Maximalwert gemessen wird, bis zu der Zeit nach Ablauf der eingestellten Zeitperiode erfasst worden sind oder nicht, und

wobei der Kennwertrechner (13) konfiguriert ist, die primäre angenäherte Gerade zu berechnen, wenn der Bestimmer bestimmt, dass alle in der Zeitzone gemessenen Herzfrequenzsignal-Informationselemente erfasst wurden.

7. Informationsverarbeitungsvorrichtung (10) nach Anspruch 2, wobei der Bestimmer (15) konfiguriert ist zum:

Bestimmen, ob die berechnete Verdauungszeitperiode gleich oder kleiner als eine Obergrenze ist oder nicht, und

Zurücksetzen der eingestellten Zeitperiode durch Vergrößern der eingestellten Zeitperiode um eine vorbestimmte Zeitperiode, wenn der Bestimmer bestimmt, dass die Verdauungszeitperiode die Obergrenze überschreitet.

8. Informationsverarbeitungsvorrichtung (10) nach Anspruch 1, ferner umfassend:

einen Änderungsratenrechner (17), der konfiguriert ist,
eine Änderungsrate der Herzfrequenz pro Zeiteinheit basierend auf mehreren durch den ersten Empfänger (11a) empfangenen Herzfrequenzsignal-Informationselementen zu berechnen, die in einer Zeitperiode von dem Ende der Mahlzeit bis zur Messung des Maximalwerts gemessen werden, zu einer Zeit, zu der die Herzfrequenz auf den Wert zur Startzeit der Mahlzeit zurückkehrt; und
einen Modellgenerator (18), der konfiguriert ist, eine Korrespondenztabelle zu erzeugen, die Zuordnungsbeziehungen zwischen der Änderungsrate der Herzfrequenz und der Verdauungsrate angibt,
wobei der Änderungsratenrechner (17) konfiguriert ist, die Änderungsrate der Herzfrequenz zu der aktuellen Zeit basierend auf einem zu einer aktuellen Zeit gemessenen Herzfrequenzsignal-Informationselement und einem oder mehreren vor der aktuellen Zeit gemessenen Herzfrequenzsignal-Informationselementen zu berechnen, und
wobei der Verdauungsratenschätzer (14) konfiguriert ist, die Verdauungsrate zu der aktuellen Zeit basierend auf der Änderungsrate der Herzfrequenz zu der aktuellen Zeit durch Referenzieren der Korrespondenztabelle zu schätzen.

9. Verdauungsratenschätzverfahren, das von einer Informationsverarbeitungsvorrichtung ausgeführt wird, die konfiguriert ist, eine Verdauungsrate von Nahrung zu schätzen, die von einem Benutzer aufgenommen wurde, der eine Herzfrequenzsignalmessvorrichtung trägt, wobei das Verdauungsratenschätzverfahren umfasst:

Empfangen, von der Herzfrequenzsignalmessvorrichtung, einer Vielzahl von Herzfrequenzsignal-Informationselementen, die in vorbestimmten Zeitintervallen gemessen wurden, und Empfangen von Informationen über eine Startzeit einer Mahlzeit und
eine Endzeit einer Mahlzeit des Benutzers von einer zu der Herzfrequenzsignalmessvorrichtung unterschiedlichen Vorrichtung;
Spezifizieren einer Herzfrequenz des Benutzers zu der Startzeit der Mahlzeit aus der Vielzahl von Herzfrequenzsignal-Informationselementen;
Berechnen, unter Verwendung der Vielzahl von Herzfrequenzsignal-Informationselementen, einer primären angenäherten Gerade, die durch Annäherung eines Profils erhalten wird, das die Abnahme einer Herzfrequenz des Benutzers angibt, die in einem Trend der Herzfrequenz nach einem Ende einer Mahlzeit enthalten ist, wobei der Trend angibt, dass die Herzfrequenz zunimmt und anschließend abnimmt;
Schätzen eines Zeitpunkts, der den gleichen Wert wie die Herzfrequenz zur Startzeit der Mahlzeit angibt, auf der berechneten primären angenäherten Gerade als die Endzeit der Verdauung, die einen Zeitpunkt angibt, zu dem die Verdauung der Nahrung endet;
Berechnen der Verdauungsrate durch Berechnen eines Verhältnisses einer Länge einer Zeitperiode von der Endzeit der Mahlzeit zu einer aktuellen Zeit in Bezug auf eine Länge einer Zeitperiode von der Endzeit der Mahlzeit zu der Endzeit der Verdauung; und
Präsentieren, dem Benutzer, von Informationen als ein Dienst gemäß der berechneten Verdauungsrate.

10. Informationsverarbeitungssystem, das konfiguriert ist, eine Verdauungsrate von Lebensmitteln, die von einem Benutzer aufgenommenen wurden, zu schätzen, wobei das Informationsverarbeitungssystem umfasst:

eine Herzfrequenzsignalmessvorrichtung (20); und
eine Informationsverarbeitungsvorrichtung (10) nach einem der Ansprüche 1 bis 8, die konfiguriert ist, mit der Herzfrequenzsignalmessvorrichtung (20) zu kommunizieren und mit dieser verbunden zu sein.

11. Verdauungsratenschätzprogramm, das, wenn es von einer Informationsverarbeitungsvorrichtung (10) ausgeführt wird, die konfiguriert ist, eine Verdauungsrate eines Benutzers, der eine Herzfrequenzsignalmessvorrichtung (20) und eine mobilen Endvorrichtung hat, zu schätzen, die Informationsverarbeitungsvorrichtung (10) veranlasst, einen Prozess gemäß Anspruch 9 auszuführen.

**Revendications**

1. Dispositif de traitement d'informations (10) configuré pour estimer un taux de digestion de nourriture ingérée par un utilisateur, le dispositif de traitement d'informations (10) comprenant :

un premier récepteur (11a) configurée pour recevoir, à partir d'un dispositif de mesure de signal de fréquence cardiaque (20) porté par l'utilisateur, une pluralité d'éléments d'informations de signal de fréquence cardiaque mesurés à des intervalles de temps prédéterminés ;

un second récepteur (11b) configurée pour recevoir des informations d'moment de début de repas et d'moment de fin de repas de l'utilisateur ;

une calculateur de quantité caractéristique (13) configuré pour :

spécifier une fréquence cardiaque de l'utilisateur à l'moment de début de repas à partir de la pluralité d'éléments d'informations de signal de fréquence cardiaque,

calculer, en utilisant la pluralité d'éléments d'information de signal de fréquence cardiaque, une ligne droite primaire d'approximation obtenue par approximation d'un profil indiquant une diminution d'une fréquence cardiaque de l'utilisateur incluse dans une tendance de la fréquence cardiaque après la fin d'un repas, la tendance indiquant que la fréquence cardiaque augmente puis diminue, et

estimer un point dans le temps indiquant la même valeur que la fréquence cardiaque à l'moment de début de repas sur la ligne droite d'approximation primaire calculée comme moment de fin de digestion indiquant un moment où la digestion de la nourriture se termine ; et

un dispositif d'estimation de taux de digestion (14) configuré pour calculer le taux de digestion en calculant un rapport d'une durée d'une période de temps entre le moment de fin de repas et un moment actuel par rapport à une durée d'une période de temps entre le moment de fin de repas et le moment de fin de digestion identifié ; et

un dispositif de présentation d'informations (16) configuré pour présenter des informations sur un service en fonction du taux de digestion calculé à l'utilisateur.

2. Dispositif de traitement d'informations (10) selon la revendication 1, comprenant en outre :

un dispositif de détermination (15) configuré pour déterminer si oui ou non le taux de digestion est égal ou supérieur à un seuil,

dans lequel le dispositif de présentation d'informations (16) est configuré pour présenter les informations sur un service à l'utilisateur lorsque le dispositif de détermination (15) détermine que le taux de digestion est égal ou supérieur au seuil.

3. Dispositif de traitement d'informations (10) selon la revendication 1, dans lequel le calculateur de quantité caractéristique (13) est configuré pour :

identifier la fréquence cardiaque au moment de début du repas et une valeur maximale de la fréquence cardiaque mesurée après la fin du repas en se référant à la pluralité d'éléments d'informations de signal de fréquence cardiaque ;

utiliser plusieurs éléments d'informations de signal de fréquence cardiaque qui sont parmi la pluralité d'éléments d'informations de signal de fréquence cardiaque et qui sont mesurés après un certain moment où la valeur maximale est mesurée, en calculant ainsi une ligne droite d'approximation primaire obtenue par approximation d'un changement de la fréquence cardiaque mesurée après le moment où la valeur maximale est mesurée ; et

utiliser la ligne droite d'approximation primaire pour identifier, comme moment de fin de digestion, un moment où la fréquence cardiaque revient à une valeur au moment de début de repas après être devenue la valeur maximale.

4. Dispositif de traitement d'informations (10) selon la revendication 3, dans lequel le calculateur de quantité caractéristique (13) est configuré pour :

définir une période de temps indiquant un moment écoulé après la mesure de la valeur maximale ; et

calculer la ligne droite d'approximation primaire en utilisant de multiples éléments d'informations de signal de fréquence cardiaque mesurés dans une période de temps à partir du moment où la valeur maximale est mesurée jusqu'à un moment après un écoulement de la période de temps définie.

5. Dispositif de traitement d'informations (10) selon la revendication 1, dans lequel le dispositif d'estimation de digestion (14) est configuré pour :

EP 3 354 193 B1

calculer une période de temps de digestion en calculant une différence entre le moment de fin de digestion et le moment de début de digestion qui est traité comme le moment de fin de repas ; et

estimer le taux de digestion en calculant un rapport d'une période de temps écoulée entre le moment de fin de repas et le moment actuel par rapport à la période de temps de digestion.

**6.** Dispositif de traitement d'informations (10) selon la revendication 2,

dans lequel le dispositif de détermination (15) est configuré pour déterminer si oui ou non tous les multiples éléments d'informations de signal de fréquence cardiaque mesurés dans une zone de temps à partir du moment où la valeur maximale est mesurée jusqu'au moment après l'écoulement de la période de temps définie ont été acquis, et

dans lequel le calculateur de quantité caractéristique (13) est configuré pour calculer la ligne droite d'approximation primaire lorsque le dispositif de détermination détermine que tous les multiples éléments d'informations de signal de fréquence cardiaque mesurés dans la zone de temps ont été acquis.

**7.** Dispositif de traitement d'informations (10) selon la revendication 2, dans lequel le dispositif de détermination (15) est configuré pour :

déterminer si oui, ou non la période de temps de digestion calculée est égale ou inférieure à une limite supérieure, et

réinitialiser la période de temps définie en augmentant la période de temps définie d'une période de temps prédéterminée lorsque le dispositif de détermination détermine que la période de temps de digestion dépasse la limite supérieure.

**8.** Dispositif de traitement d'informations (10) selon la revendication 1, comprenant en outre :

un calculateur de taux de changement (17) configuré pour calculer un taux de changement de la fréquence cardiaque par unité de temps sur la base de multiples éléments d'informations de signal de fréquence cardiaque, reçus par le premier récepteur (11a), mesurés dans une période de temps à partir de la fin de repas, à la mesure de la valeur maximale, à un moment où la fréquence cardiaque revient à la valeur au moment de début de repas ; et

un générateur de modèle (18) configuré pour générer une table de correspondance indiquant des relations d'association entre le taux de changement de la fréquence cardiaque et le taux de digestion,

dans lequel le calculateur de taux de changement (17) est configuré pour calculer le taux de changement de la fréquence cardiaque au moment actuel sur la base d'un élément d'informations de signal de fréquence cardiaque mesuré à un moment actuel et d'un ou plusieurs éléments d'informations de signal de fréquence cardiaque mesurés avant le moment actuel, et

dans lequel le dispositif d'estimation de taux de digestion (14) est configuré pour estimer le taux de digestion au moment actuel sur la base du taux de changement de la fréquence cardiaque au moment actuel en se référant à la table de correspondance.

**9.** Procédé d'estimation du taux de digestion exécuté par un dispositif de traitement d'informations configuré pour estimer un taux de digestion de nourriture ingérée par un utilisateur portant un dispositif de mesure de signal de fréquence cardiaque, le procédé d'estimation de taux de digestion comprenant les étapes consistant à :

recevoir, à partir du dispositif de mesure de signal de fréquence cardiaque, une pluralité d'éléments d'informations de signal de fréquence cardiaque mesurés à des intervalles de temps prédéterminés et recevoir des informations de moment de début de repas et de moment de fin de repas de l'utilisateur à partir d'un dispositif différent du dispositif de mesure de signal de fréquence cardiaque ;

spécifier une fréquence cardiaque de l'utilisateur au moment de début de repas à partir de la pluralité d'éléments d'informations de signal de fréquence cardiaque ;

calculer, en utilisant la pluralité d'éléments d'informations de signal de fréquence cardiaque, une ligne droite d'approximation primaire obtenue par approximation d'un profil indiquant une diminution d'une fréquence cardiaque de l'utilisateur incluse dans une tendance de la fréquence cardiaque après une fin d'un repas, la tendance indiquant que la fréquence cardiaque augmente puis diminue ;

estimer un point dans le temps indiquant la même valeur que la fréquence cardiaque au moment de début de repas sur la ligne droite d'approximation primaire calculée comme moment de fin de digestion indiquant un moment où la digestion de la nourriture se termine ;

calculer le taux de digestion en calculant un rapport d'une durée d'une période de temps entre le moment de fin de repas et un moment actuel par rapport à une durée d'une période de temps entre le moment de fin de repas et le moment de fin de digestion ; et

présenter à l'utilisateur des informations sur un service en fonction du taux de digestion calculé.

**10.** Système de traitement d'informations configuré pour estimer un taux de digestion de nourriture ingérée par un utilisateur, le système de traitement d'informations comprenant :

un dispositif de mesure de signal de fréquence cardiaque (20) ; et

un dispositif de traitement d'informations (10) selon l'une quelconque des revendications 1 à 8, configuré pour communiquer avec et couplé au dispositif de mesure de signal de fréquence cardiaque (20).

**11.** Programme d'estimation de taux de digestion qui, lorsqu'il est exécuté par un dispositif de traitement d'informations (10) configuré pour estimer un taux de digestion d'un utilisateur ayant un dispositif de mesure de signal de fréquence cardiaque (20) et un dispositif de terminal mobile, amène le dispositif de traitement d'informations (10) à exécuter un processus selon la revendication 9.

# FIG. 1

100

# FIG. 2

```
                                                                    ⌐10
┌──────────────────────────────────────────────────────────────────────┐
│                                  INFORMATION PROCESSING DEVICE         │
│     ⌐61            ⌐65                           ⌐66                    │
│  ┌────────┐   ┌──────────────┐         ┌──────────────┐                │
│  │  CPU   │   │ INPUT DEVICE │         │   DISPLAY    │                │
│  │        │   │              │         │   DEVICE     │                │
│  └───┬────┘   └──────┬───────┘         └──────┬───────┘                │
│      │               │        ⌐70             │                        │
│──────┴───────────────┴────────────────┬───────┴───────────┬────────────│
│     ⌐62            ⌐63          ⌐64    │    ⌐67            │   ⌐68      │
│  ┌────────┐   ┌──────────┐  ┌──────────┐  ┌──────────┐  ┌──────────┐   │
│  │  ROM   │   │   RAM    │  │ STORAGE  │  │ NETWORK  │  │ PORTABLE │   │
│  │        │   │          │  │ DEVICE   │  │INTERFACE │  │ STORAGE  │   │
│  └────────┘   └──────────┘  └──────────┘  └──────────┘  │MEDIUM DRIVE│ │
│                                                         └──────────┘   │
└──────────────────────────────────────────────────────────────────────┘
                                                              ⌐69
                                                         ┌──────────┐
                                                         │ PORTABLE │
                                                         │ STORAGE  │
                                                         │ MEDIUM   │
                                                         └──────────┘
```

# FIG. 3

MEAL START TIME    MEAL END TIME

y

HEART RATE [bpm]

0    240    x

TIME [min]

# FIG. 4

START

RECEIVE MULTIPLE HEART RATE SIGNAL
INFORMATION ITEMS FROM HEART RATE SIGNAL
MEASURING DEVICE — S101

ACQUIRE INFORMATION OF MEAL START TIME AND
MEAL END TIME — S102

IDENTIFY HEART RATE AT MEAL START TIME — S103

IDENTIFY PEAK VALUE OF HEART RATE AFTER
END OF MEAL — S104

S105
IS PRIMARY
NO — APPROXIMATE STRAIGHT LINE TO BE
CALCULATED?

YES

CALCULATE PRIMARY APPROXIMATE STRAIGHT LINE — S106

ESTIMATE DIGESTION END TIME — S107

CALCULATE TIME PERIOD FROM MEAL END TIME TO
MEASUREMENT TIME OF PEAK VALUE — S108

TO S109

# FIG. 5

12a

| MEASUREMENT TIME [hh:mm] | HEART RATE [bpm] |
|---|---|
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |

# FIG. 6

| MEASUREMENT TIME [hh:mm] | HEART RATE [bpm] |
|---|---|
| 12:00 | 80 |
| 12:01 | 80 |
| 12:02 | 80 |
| 12:03 | 90 |
| 12:04 | 100 |
| 12:05 | 120 |
| 12:06 | 100 |
| 12:07 | 90 |
| 12:08 | 80 |
| 12:09 | 84 |
| 12:10 | 86 |
| 12:11 | 88 |
| 12:12 | 90 |
| 12:13 | 92 |
| ... | ... |
| 12:20 | 90 |
| ... | ... |
| 12:25 | 100 |
| ... | ... |
| 12:35 | 99 |
| ... | ... |
| 12:45 | 98 |
| ... | ... |

12a

# FIG. 7

| MEAL ID | MEAL START TIME [hh:mm] | MEAL END TIME [hh:mm] |
|---------|-------------------------|-----------------------|
|         |                         |                       |
|         |                         |                       |
|         |                         |                       |

12b

# FIG. 8

12b

| MEAL ID | MEAL START TIME [hh:mm] | MEAL END TIME [hh:mm] |
|---------|------------------------|----------------------|
| 1 | 12:02 | 12:07 |
| 2 | | |
| ... | | |

FIG. 9

5 12c

| MEAL ID | CHARACTERISTIC AMOUNT 1<br><br>HEART RATE AT MEAL START TIME [bpm] | CHARACTERISTIC AMOUNT 2<br><br>PEAK VALUE [bpm] | CHARACTERISTIC AMOUNT 3<br><br>PRIMARY APPROXIMATE STRAIGHT LINE ax+b | CHARACTERISTIC AMOUNT 4<br><br>DIGESTION END TIME [hh:mm] | CHARACTERISTIC AMOUNT 5<br><br>TIME PERIOD FROM MEAL END TIME TO MEASUREMENT TIME OF PEAK VALUE [min] | CHARACTERISTIC AMOUNT 6<br><br>TIME PERIOD FROM MEASUREMENT TIME OF PEAK VALUE TO DIGESTION END TIME [min] |
|---|---|---|---|---|---|---|
| 1 | | | | | | |
| 2 | | | | | | |
| ... | | | | | | |

FIG. 10

| MEAL ID | CHARACTERISTIC AMOUNT 1 HEART RATE AT MEAL START TIME [bpm] | CHARACTERISTIC AMOUNT 2 PEAK VALUE [bpm] | CHARACTERISTIC AMOUNT 3 PRIMARY APPROXIMATE STRAIGHT LINE ax+b | CHARACTERISTIC AMOUNT 4 DIGESTION END TIME [hh:mm] | CHARACTERISTIC AMOUNT 5 TIME PERIOD FROM MEAL END TIME TO MEASUREMENT TIME OF PEAK VALUE [min] | CHARACTERISTIC AMOUNT 6 TIME PERIOD FROM MEASUREMENT TIME OF PEAK VALUE TO DIGESTION END TIME [min] |
|---|---|---|---|---|---|---|
| 1 | 80 | | | | | |
| 2 | | | | | | |
| ... | | | | | | |

12c

## FIG. 11

_12c

| MEAL ID | CHARACTERISTIC AMOUNT 1 | CHARACTERISTIC AMOUNT 2 | CHARACTERISTIC AMOUNT 3 | CHARACTERISTIC AMOUNT 4 | CHARACTERISTIC AMOUNT 5 | CHARACTERISTIC AMOUNT 6 |
|---|---|---|---|---|---|---|
| | HEART RATE AT MEAL START TIME [bpm] | PEAK VALUE [bpm] | PRIMARY APPROXIMATE STRAIGHT LINE ax+b | DIGESTION END TIME [hh:mm] | TIME PERIOD FROM MEAL END TIME TO MEASUREMENT TIME OF PEAK VALUE [min] | TIME PERIOD FROM MEASUREMENT TIME OF PEAK VALUE TO DIGESTION END TIME [min] |
| 1 | 80 | 100 | | | | |
| 2 | | | | | | |
| ... | | | | | | |

# FIG. 12

DIGESTION TIME PERIOD

CHARACTERISTIC AMOUNT 5

CHARACTERISTIC AMOUNT 6

y

MEAL END TIME

MEAL START TIME

HEART RATE [bpm]

PRIMARY APPROXIMATE STRAIGHT LINE
y=ax+b

(CHARACTERISTIC AMOUNT 3)

c

0

x

y=c

CURRENT TIME

DIGESTION END TIME
(CHARACTERISTIC AMOUNT 4)

CHARACTERISTIC AMOUNT 2

CHARACTERISTIC AMOUNT 1

TIME [min]

FIG. 13

12c

| MEAL ID | CHARACTERISTIC AMOUNT 1 HEART RATE AT MEAL START TIME [bpm] | CHARACTERISTIC AMOUNT 2 PEAK VALUE [bpm] | CHARACTERISTIC AMOUNT 3 PRIMARY APPROXIMATE STRAIGHT LINE ax+b | CHARACTERISTIC AMOUNT 4 DIGESTION END TIME [hh:mm] | CHARACTERISTIC AMOUNT 5 TIME PERIOD FROM MEAL END TIME TO MEASUREMENT TIME OF PEAK VALUE [min] | CHARACTERISTIC AMOUNT 6 TIME PERIOD FROM MEASUREMENT TIME OF PEAK VALUE TO DIGESTION END TIME [min] |
|---|---|---|---|---|---|---|
| 1 | 80 | 100 | -0.1x+100 | | | |
| 2 | | | | | | |
| ... | | | | | | |

# FIG. 14

5 12c

| MEAL ID | CHARACTERISTIC AMOUNT 1 | CHARACTERISTIC AMOUNT 2 | CHARACTERISTIC AMOUNT 3 | CHARACTERISTIC AMOUNT 4 | CHARACTERISTIC AMOUNT 5 | CHARACTERISTIC AMOUNT 6 |
|---|---|---|---|---|---|---|
| | HEART RATE AT MEAL START TIME [bpm] | PEAK VALUE [bpm] | PRIMARY APPROXIMATE STRAIGHT LINE ax+b | DIGESTION END TIME [hh:mm] | TIME PERIOD FROM MEAL END TIME TO MEASUREMENT TIME OF PEAK VALUE [min] | TIME PERIOD FROM MEASUREMENT TIME OF PEAK VALUE TO DIGESTION END TIME [min] |
| 1 | 80 | 100 | -0.1x+100 | 15:45 | | 200 |
| 2 | | | | | | |
| ... | | | | | | |

# FIG. 15

<p align="right">12c</p>

| MEAL ID | CHARACTERISTIC AMOUNT 1 | CHARACTERISTIC AMOUNT 2 | CHARACTERISTIC AMOUNT 3 | CHARACTERISTIC AMOUNT 4 | CHARACTERISTIC AMOUNT 5 | CHARACTERISTIC AMOUNT 6 |
|---|---|---|---|---|---|---|
| | HEART RATE AT MEAL START TIME [bpm] | PEAK VALUE [bpm] | PRIMARY APPROXIMATE STRAIGHT LINE ax+b | DIGESTION END TIME [hh:mm] | TIME PERIOD FROM MEAL END TIME TO MEASUREMENT TIME OF PEAK VALUE [min] | TIME PERIOD FROM MEASUREMENT TIME OF PEAK VALUE TO DIGESTION END TIME [min] |
| 1 | 80 | 100 | -0.1x+100 | 15:45 | 18 | 200 |
| 2 | | | | | | |
| ... | | | | | | |

# FIG. 16

S108

S109

IS CURRENT TIME
WHEN DIGESTION RATE IS TO BE
ESTIMATED?

NO

YES

S110

CALCULATE DIGESTION RATE AT
CURRENT TIME

S111

IS INFORMATION TO BE
PRESENTED TO USER?

NO

YES

S112

IS SERVICE BEING
CONTINUOUSLY PROVIDED?

YES

NO

S113

PRESENT INFORMATION TO USER

END

# FIG. 17

12d

| CALCULATION TIME [hh:mm] | DIGESTION RATE [%] |
|---|---|
| ... | |
| 12:30 | |
| 12:40 | |
| 12:50 | |
| 13:00 | |
| 13:10 | |
| 13:20 | |
| 13:30 | |
| 13:40 | |
| 13:50 | |
| 14:00 | |
| 14:10 | |
| 14:20 | |
| 14:30 | |
| ... | |

# FIG. 18

12d

| CALCULATION TIME [hh:mm] | DIGESTION RATE [%] |
|---|---|
| ... | |
| 12:30 | 11 |
| 12:40 | |
| 12:50 | |
| 13:00 | |
| 13:10 | |
| 13:20 | |
| 13:30 | |
| 13:40 | |
| 13:50 | |
| 14:00 | |
| 14:10 | |
| 14:20 | |
| 14:30 | |
| ... | |

# FIG. 19

12d

| CALCULATION TIME [hh:mm] | DIGESTION RATE [%] |
|:---:|:---:|
| ... | |
| 12:30 | 11 |
| 12:40 | 15 |
| 12:50 | 20 |
| 13:00 | 24 |
| 13:10 | 29 |
| 13:20 | 33 |
| 13:30 | 38 |
| 13:40 | 43 |
| 13:50 | 47 |
| 14:00 | 52 |
| 14:10 | 56 |
| 14:20 | 61 |
| 14:30 | 66 |
| ... | |

# FIG. 20

```
          ┌─────────┐
          │  S108   │
          └────┬────┘
               │
               ▼          S108a
          ◇─────────────◇          NO
          DIGESTION TIME PERIOD ──────────────┐
          ≦UPPER LIMIT?                        │
          ◇─────────────◇                      ▼              S108b
               │ YES                  ┌──────────────────────┐
               │                      │ RESET SET TIME PERIOD BY │
               │                      │ INCREASING SET TIME PERIOD BY │
               │                      │ PREDETERMINED TIME PERIOD │
               │                      └───────────┬──────────┘
               │                                  │
               │                                  ▼
               │                            RETURN TO S106
               ▼          S109
  ┌───────◇─────────────◇
  │  NO   IS CURRENT
  ◄───────TIME WHEN DIGESTION RATE
  │       IS TO BE ESTIMATED?
  │       ◇─────────────◇
  │            │ YES
  │            ▼
  │  ┌──────────────────────┐
  │  │ CALCULATE DIGESTION RATE AT │  S110
  │  │ CURRENT TIME          │
  │  └───────────┬──────────┘
  │              │
  │              ▼          S111
  │    NO  ◇─────────────◇
  │  ┌─────IS INFORMATION TO BE
  │  │     PRESENTED TO USER?
  │  │     ◇─────────────◇
  │  ▼          S112         │ YES
  │ ◇─────────────◇          │
  │ IS SERVICE BEING         │
YES│ CONTINUOUSLY            │
  └─│ PROVIDED?              │
    ◇─────────────◇          │
         │ NO                │
         │                   ▼
         │        ┌──────────────────────┐
         │        │ PRESENT INFORMATION TO USER │  S113
         │        └───────────┬──────────┘
         │                    │
         └────────────────────┤
                              ▼
                        ┌─────────┐
                        │   END   │
                        └─────────┘
```

# FIG. 21

# FIG. 22

START

RECEIVE MULTIPLE HEART RATE SIGNAL INFORMATION ITEMS FROM HEART RATE SIGNAL MEASURING DEVICE — S201

ACQUIRE INFORMATION OF MEAL START TIME AND MEAL END TIME — S202

IDENTIFY HEART RATE AT MEAL START TIME — S203

IDENTIFY PEAK VALUE OF HEART RATE AFTER END OF MEAL — S204

S205
IS PRIMARY APPROXIMATE STRAIGHT LINE TO BE CALCULATED?
NO
YES

CALCULATE PRIMARY APPROXIMATE STRAIGHT LINE — S206

ESTIMATE DIGESTION END TIME — S207

CALCULATE TIME PERIOD FROM MEAL END TIME TO MEASUREMENT TIME OF PEAK VALUE — S208

TO S209

# FIG. 23

```
        ┌──────────┐
        │   S208   │
        └────┬─────┘
             │
             ▼                              S209
NO  ◄─────────────◄  IS CURRENT TIME
              WHEN DIGESTION RATE IS TO BE
                      ESTIMATED?
                         │ YES
                         ▼                  S210
            ┌─────────────────────────┐
            │ CALCULATE DIGESTION RATE AT │
            │      CURRENT TIME        │
            └────────────┬────────────┘
                         │
                         ▼                  S211
        NO  ◄─────────  IS INFORMATION TO BE
                        PRESENTED TO USER?
                         │ YES
             S212        │
   YES ◄── IS SERVICE BEING              S213
        CONTINUOUSLY PROVIDED?   ┌─────────────────────────┐
                 │ NO           │ TRANSMIT INSTRUCTION SIGNAL │
                 │              │ INDICATING THAT INFORMATION IS TO BE │
                 │              │   PRESENTED TO USER      │
                 │              └────────────┬────────────┘
                 │                           │
                 ▼                           ▼
                            ┌──────────┐
                            │   END    │
                            └──────────┘
```

# FIG. 24

300

HEART RATE SIGNAL MEASURING DEVICE — 20

WIRELESS DEVICE — 40

WIRELESS DEVICE — 50

10c

RECEIVER — 11

FIRST RECEIVER — 11a

SECOND RECEIVER — 11b

DIGESTION RATE ESTIMATOR — 14

CHARACTERISTIC AMOUNT CALCULATOR — 13

DETERMINER — 15

INFORMATION PRESENTER — 16

STORAGE SECTION — 12

INFORMATION PROCESSING DEVICE

30

HEART RATE MANAGEMENT TABLE — 12a

MEAL TIME MANAGEMENT TABLE — 12b

CHARACTERISTIC AMOUNT MANAGEMENT TABLE — 12c

DIGESTION RATE ESTIMATION MANAGEMENT TABLE — 12d

STORAGE DEVICE

# FIG. 25

400

20

HEART RATE SIGNAL
MEASURING DEVICE

10d

11a

FIRST RECEIVER

11b

SECOND RECEIVER

RECEIVER

11

13

CHARACTERISTIC
AMOUNT
CALCULATOR

14

DIGESTION RATE
ESTIMATOR

15

DETERMINER

16

INFORMATION
PRESENTER

17

CHANGE RATE
CALCULATOR

18

MODEL GENERATOR

12a

HEART RATE
MANAGEMENT TABLE

12b

MEAL TIME
MANAGEMENT TABLE

12c

CHARACTERISTIC
AMOUNT
MANAGEMENT TABLE

12d

DIGESTION RATE
ESTIMATION
MANAGEMENT TABLE

STORAGE SECTION

12

INFORMATION PROCESSING DEVICE

# FIG. 26

```
                    ┌─────────┐
                    │  START  │
                    └────┬────┘
                         ▼
┌──────────────────────────────────────────┐
│    RECEIVE MULTIPLE HEART RATE SIGNAL     │
│  INFORMATION ITEMS FROM HEART RATE SIGNAL │ ～ S401
│             MEASURING DEVICE              │
└──────────────────┬───────────────────────┘
                   ▼
┌──────────────────────────────────────────┐
│ ACQUIRE INFORMATION OF MEAL START TIME, MEAL │ ～ S402
│      END TIME, AND DETAILS OF MEAL        │
└──────────────────┬───────────────────────┘
                   ▼
┌──────────────────────────────────────────┐
│     IDENTIFY HEART RATE AT MEAL START TIME │ ～ S403
└──────────────────┬───────────────────────┘
                   ▼
        ┌──────────────────────────┐ S404
  NO    ╱  HAS HEART RATE RETURNED TO ╲
 ◄──────┤   VALUE AT MEAL START TIME?   │
        ╲                            ╱
         └────────────┬─────────────┘
                   │ YES
                   ▼
┌──────────────────────────────────────────┐
│         IDENTIFY DIGESTION END TIME        │ ～ S405
└──────────────────┬───────────────────────┘
                   ▼
┌──────────────────────────────────────────┐
│       IDENTIFY DIGESTION TIME PERIOD       │ ～ S406
└──────────────────┬───────────────────────┘
                   ▼
┌──────────────────────────────────────────┐
│  GENERATE CHANGE RATE AND DIGESTION RATE   │ ～ S407
│          CORRESPONDENCE TABLE              │
└──────────────────┬───────────────────────┘
                   ▼
              ┌─────────┐
              │ TO S408 │
              └─────────┘
```

# FIG. 27

| RATE OF CHANGE OF HEART RATE [bpm] | DIGESTION RATE [%] |
|:---:|:---:|
| 0.29 | 10 |
| 0.12 | 20 |
| -0.01 | 30 |
| -0.03 | 40 |
| -0.06 | 50 |
| -0.07 | 60 |
| -0.08 | 70 |
| -0.08 | 80 |
| -0.02 | 90 |
| 0.00 | 100 |

# FIG. 28

# FIG. 29

```
                          ( S407 )
                             |
                             |<---------------------------+
                             v                            |
                    ┌─────────────────────┐  S408         |
                    │  RECEIVE HEART RATE  │               |
                    │ SIGNAL INFORMATION   │               |
                    │  ITEMS FROM HEART    │               |
                    │ RATE SIGNAL          │               |
                    │ MEASURING DEVICE     │               |
                    └─────────────────────┘               |
                             |                            |
                             v                            |
                    ┌─────────────────────┐  S409         |
                    │ ACQUIRE INFORMATION  │               |
                    │ OF MEAL START TIME   │               |
                    │  AND MEAL END TIME   │               |
                    └─────────────────────┘               |
                             |                            |
                             v                            |
                    ┌─────────────────────┐  S410         |
                    │ CALCULATE RATE OF    │               |
                    │ CHANGE OF HEART RATE │               |
                    │   AT CURRENT TIME    │               |
                    └─────────────────────┘               |
                             |                            |
                             v                            |
                    ┌─────────────────────┐  S411         |
                    │ REFERENCE CHANGE     │               |
                    │ RATE AND DIGESTION   │               |
                    │ RATE CORRESPONDENCE  │               |
                    │ TABLE AND ESTIMATE   │               |
                    │ DIGESTION RATE AT    │               |
                    │ CURRENT TIME         │               |
                    └─────────────────────┘               |
```

RECEIVE HEART RATE SIGNAL INFORMATION ITEMS FROM HEART RATE SIGNAL MEASURING DEVICE — S408

ACQUIRE INFORMATION OF MEAL START TIME AND MEAL END TIME — S409

CALCULATE RATE OF CHANGE OF HEART RATE AT CURRENT TIME — S410

REFERENCE CHANGE RATE AND DIGESTION RATE CORRESPONDENCE TABLE AND ESTIMATE DIGESTION RATE AT CURRENT TIME — S411

S412 — IS INFORMATION TO BE PRESENTED TO USER?
NO
YES

S413 — IS SERVICE BEING CONTINUOUSLY PROVIDED?
YES
NO

S414 — PRESENT INFORMATION TO USER

END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10504739 W
- JP 2004000138 A
- JP 2009201805 A
- JP 2011115508 A

- JP 2008061790 A
- US 5398688 A
- US 2006064037 A
- JP 2008761790 B

**Non-patent literature cited in the description**

- **OUCHI, K. et al.** LifeMinder: a wearable healthcare support system with timely instruction based on the user's context. *The 8th IEEE International Workshop on Advanced Motion Control,* 2004, 445-450